# EUROPEAN PATENT APPLICATION

(11) **EP 4 726 042 A2**
(43) Date of publication of application: **15.04.2026**
(21) Application number: 25203492.1
(22) Date of filing: 19.09.2025
(51) Int. Cl.: C12N 15/113

(54) **ANTISENSE OLIGOMERS TARGETING SETD5 FOR UPREGULATION AND USE THEREOF**

(30) Priority: 19.09.2024 KR 20240126792
(71) Applicant: Korea Advanced Institute of Science and Technology, Daejeon 34141 (KR)
(72) Inventor: KIM, Jinkuk, Daejeon (KR); KIM, Zion, Daejeon (KR)
(74) Representative: Appleyard Lees IP LLP

(57) **Abstract**

Disclosed herein are an antisense oligonucleotide that targets a 5'-UTR of SETD5 to induce upregulation of SETD5 expression, and its use in the treatment of diseases associated with SETD5 deficiency. The antisense oligonucleotide targeting the 5'-UTR of SETD5 induces splicing to increase the production of a productive isoform mRNA that does not include regions that inhibit translation of SETD5, thereby increasing the expression level of SETD5, and enabling the treatment or amelioration of diseases or symptoms caused by deficiency of SETD5 in a subject.

## Description

### Technical Field

Disclosed herein are an antisense oligonucleotide that targets the pre-mRNA of the human *SETD5* (SET domain containing 5) to induce upregulation of SETD5 expression, and its use in the treatment of diseases associated with functional SETD5 deficiency. The antisense oligonucleotide targeting the pre-mRNA of the human *SETD5* gene induces splicing to increase the production of a productive isoform mRNA that does not include regions that inhibit SETD5 translation, thereby increasing the expression level of SETD5, and enabling the treatment or amelioration of diseases or symptoms caused by deficiency of functional SETD5 in a subject.

The present research was supported by Samsung Science & Technology Foundation (Project No: SRFC-MA2102).

### Background Art

Many genetic disorders arise when one of the two copies of a gene inherited from the parents becomes modified due to a mutation. As a result, the normal amount of the corresponding protein cannot be produced. Therefore, increasing protein production from the remaining unmodified, normal copy may serve as a new strategy for treating genetic disorders. A messenger RNA (mRNA) produced by transcription of a gene contains translational regulatory elements that control the efficiency of protein production, and such elements may serve as potential targets for enhancing protein expression from the normal gene.

An antisense oligonucleotide (ASO) binds to a pre-mRNA or mRNA with a complementary sequence and modulates RNA processing. Depending on its design, an ASO can alter the splicing pattern of a pre-mRNA to generate a different protein isoform, or it can induce degradation of the mRNA to inhibit protein production. In eukaryotic cells, pre-mRNA undergoes splicing to produce mature mRNA isoforms. This is an important process in post-transcriptional gene regulation. Splicing can be classified into constitutive splicing, which removes introns and joins exons to form a single mRNA, and alternative splicing, which selectively includes or excludes specific exons to generate diverse mRNA isoforms. Alternative splicing is particularly prominent in 5'-untranslated regions (5'UTRs) of mammalian genes. Compared to coding regions, the 5'UTR is subjected to relatively fewer constraints related to protein structure, resulting in a higher proportion of alternative transcript isoforms.

SETD5 (SET domain containing 5) syndrome is a rare disorder caused by mutations in the *SETD5* gene, which is located on chromosome 3. The *SETD5* gene plays an important role in gene expression, and mutations in this gene can have a profound impact on neurodevelopment and growth. This syndrome primarily results from haploinsufficiency, condition in which a mutation in the *SETD5* gene leads to insufficient production of the normal *SETD5* gene product. Representative symptoms of SETD5 syndrome include intellectual disability, delayed speech development, hypotonia, and facial dysmorphisms, and some patients may exhibit behavioral problems similar to those observed in autism spectrum disorder. In rare cases, it may also be accompanied by feeding difficulties or growth retardation. In most cases, this disease is not inherited from the parents and arises from a de novo mutation in the *SETD5* gene. This disease was first reported in 2014, and is estimated to occur in 1 in 1 million people worldwide. However, recent studies have identified *SETD5* gene mutations in about 0.7 % of patients with intellectual disability, suggesting that there may be a higher number of undiagnosed cases. To date, there is no effective treatment for SETD5 syndrome.

Studies on antisense oligomers (ASOs) for the treatment of diseases caused by insufficient expression of functional proteins are actively underway, including methods for promoting constitutive splicing using ASOs targeting introns that are not removed due to incomplete splicing (see US Patent Application Publication No. 2019-0070213). However, there remains a need for antisense oligonucleotide therapeutics that increase gene product levels by inducing alternative splicing to elevate the production of mRNA isoforms with high translation efficiency.

In this regard, the inventors have identified target regions in the pre-mRNA of SETD5 capable of inducing upregulation of SETD5 expression, and have developed antisense oligonucleotides that promote alternative splicing of these regions.

### Description of Disclosure

### Technical Problem

The present disclosure aims to identify target regions for increasing the production of normal SETD5 protein for the treatment of SETD5 syndrome, and to provide antisense oligonucleotides that enhance the expression level of SETD5 protein by promoting alternative splicing to generate productive SETD5 isoforms.

The present disclosure also aims to provide a method for treating diseases associated with abnormal level and/or activity of SETD5 by using antisense oligonucleotides that increase SETD5 protein expression by regulating alternative splicing to promote the production of productive SETD5 isoforms.

### Solution to Problem

An aspect of the present disclosure provides an antisense oligonucleotide targeting a pre-mRNA of the human *SETD5* gene,
wherein the antisense oligonucleotide comprises 13 to 40 consecutive nucleotides, and binds to *SETD5* pre-mRNA by hybridization to increase the expression of SETD5.

In an embodiment of the present disclosure, the antisense oligonucleotide targeting a pre-mRNA of the human *SETD5* gene comprises 13 to 40 consecutive nucleotides and binds to a target region of the SETD5 pre-mRNA by hybridization via at least 13 consecutive Watson-Crick base pairings (A:T or G:C) or wobble base pairings (G:U, I:A, I:C, or I:U),
wherein the target region comprises a sequence selected from the group consisting of SEQ ID NOs: 1 to 15, 17 to 19, 47 to 68, 95 to 97, 100, 121 to 124, 126, 131 to 133, 137, and 138.

The target regions of the antisense oligonucleotide that induce upregulation of SETD5 expression were identified by determining the regions of *SETD5* pre-mRNA that modulate its translational activity. For this purpose, polysome profiling analysis and translation activity prediction using a translation prediction model were performed. Specifically, polysome profiling in human neuronal cells revealed that the number of ribosomes bound to mature mRNA varies depending on the inclusion of a specific exon within the 5'-UTR, indicating corresponding differences in SETD5 translational activity. Furthermore, the Framepool model (PLoS Comput Biol 17(5):e1008982, PMID: 33970899), which predicts the average number of ribosomes bound per mRNA based on a given 5'UTR sequence was utilized to predict and compare the translational activity of each transcript isoform of SETD5 annotated in the human RefSeq. As a result, the model predicted that the number of ribosomes bound per mRNA varies depending on the inclusion of a specific exon in the mature mRNA, indicating corresponding differences in SETD5 translational activity. Through this approach, it was found that splicing events, resulting in the exclusion of SETD5 exon 3a or 3b or both simultaneously, enhance SETD5 translational activity and allow the production of a full-length protein. Therefore, in the present disclosure, exons 3a and 3b were identified as target exons. Within the 5'-UTR of the *SETD5* gene, exon 3a is located on the plus strand of chr3:9,429,827-9,430,051 (hg38) (SEQ ID NO: 173), and exon 3b is located on the plus strand of chr3:9,433,370-9,433,562 (hg38) (SEQ ID NO: 174). Antisense oligonucleotides were designed to target a sequence selected from the group consisting of SEQ ID NOs: 1 to 20 and 91 to 104 within the 5'-UTR target region. These antisense oligonucleotides were shown to induce splicing in a manner that prevents the inclusion of translational inhibitory regions of the pre-mRNA in the mature mRNA, thereby increasing the levels of productive isoform mRNA and consequently enhancing SETD5 expression. Antisense oligonucleotides were further designed to target sequences selected from the group consisting of SEQ ID NOs: 47 to 68 and 121 to 140 within the target region of antisense oligonucleotides that were found to have high activity in increasing the production of productive SETD5 isoform mRNAs and enhancing SETD5 expression, and these antisense oligonucleotides were screened for their ability to increase SETD5 expression.

In an embodiment of the present disclosure, the antisense oligonucleotide comprises a sequence of consecutive nucleotides that is at least 80 %, for example, at least 85 %, at least 90 %, at least 91 %, at least 92 %, at least 93 %, at least 94 %, at least 95 %, at least 96 %, at least 97 %, at least 98 %, or 100 % complementary to a target region of a sequence selected from the group consisting of SEQ ID NOs: 1 to 15, 17 to 19, 47 to 68, 95 to 97, 100, 121 to 124, 126, 131 to 133, 137, and 138.

In an embodiment of the present disclosure, the antisense oligonucleotide may be 14 to 30, 15 to 25, 17 to 24, or 18 to 20 nucleotides in length, without being limited thereto.

In an embodiment of the present disclosure, the antisense oligonucleotide may be completely complementary (100 % complementary) to a target region having a sequence selected from the group consisting of SEQ ID NOs: 1 to 15, 17 to 19, 47 to 68, 95 to 97, 100, 121 to 124, 126, 131 to 133, 137, and 138, or may comprise mismatches, for example, one, two, or three or more consecutive mismatches, to an extent that allows binding to the target region by hybridization via at least 13 consecutive Watson-Crick base pairings or wobble base pairings, thereby increasing the expression of SETD5.

In an embodiment of the present disclosure, the target region may comprise a sequence selected from the group consisting of SEQ ID NOs: 2 to 7, 9, 11, 12, 48, 53, 55, 60, 65 to 67, 97, 121 to 123, 132, and 133.

In an embodiment of the present disclosure, the target region may comprise a sequence selected from the group consisting of SEQ ID NOs: 2 to 7, 48, 53, 55, 65, 97, 122, 123, 132, and 133.

In an embodiment of the present disclosure, the target region may comprise a sequence selected from the group consisting of SEQ ID NOs: 2, 53, 65, 97, 123, 132, and 133.

In an embodiment of the present disclosure, the antisense oligonucleotide may comprise a sequence selected from the group consisting of SEQ ID NOs: 22 to 36, 38 to 40, 69 to 90, 110 to 112, 115, 142 to 145, 147, 152 to 154, 158, and 159.

In an embodiment of the present disclosure, the antisense oligonucleotide may comprise a sequence selected from the group consisting of SEQ ID NOs: 23 to 28, 30, 32, 33, 70, 75, 77, 82, 87 to 89, 112, 142 to 144, 153, and 154.

In an embodiment of the present disclosure, the antisense oligonucleotide may comprise a sequence selected from the group consisting of SEQ ID NOs: 23 to 28, 70, 75, 77, 87, 112, 143, 144, 153, and 154.

In an embodiment of the present disclosure, the antisense oligonucleotide may comprise a sequence selected from the group consisting of SEQ ID NOs: 23, 75, 87, 112, and 144.

In an embodiment of the present disclosure, the antisense oligonucleotide targeting a pre-mRNA of the human SETD5 gene comprises 13 to 40 consecutive nucleotides, and binds to 80 % or more of nucleobases in a target region of the *SETD5* pre-mRNA by hybridization via Watson-Crick base pairings (A:T or G:C) or wobble base pairings (G:U, I:A, I:C, or I:U), to increase SETD5 expression,
wherein the target region comprises a sequence selected from the group consisting of SEQ ID NOs: 1 to 15, 17 to 19, 47 to 68, 95 to 97, 100, 121 to 124, 126, 131 to 133, 137, and 138.

In an embodiment of the present disclosure, the target region may be a sequence selected from the group consisting of SEQ ID NOs: 2 to 7, 9, 11, 12, 48, 53, 55, 60, 65 to 67, 97, 121 to 123, 132, and 133.

In an embodiment of the present disclosure, the target region may be a sequence selected from the group consisting of SEQ ID NOs: 2 to 7, 48, 53, 55, 65, 97, 122, 123, 132, and 133.

In an embodiment of the present disclosure, the target region may be a sequence selected from the group consisting of SEQ ID NOs: 2, 53, 65, 97, 123, 132, and 133.

In an embodiment of the present disclosure, the antisense oligonucleotide may be a sequence selected from the group consisting of SEQ ID NOs: 22 to 36, 38 to 40, 69 to 90, 110 to 112, 115, 142 to 145, 147, 152 to 154, 158, and 159.

In an embodiment of the present disclosure, the antisense oligonucleotide may be a sequence selected from the group consisting of SEQ ID NOs: 23 to 28, 30, 32, 33, 70, 75, 77, 82, 87 to 89, 112, 142 to 144, 153, and 154.

In an embodiment of the present disclosure, the antisense oligonucleotide may be a sequence selected from the group consisting of SEQ ID NOs: 23 to 28, 70, 75, 77, 87, 112, 143, 144, 153, and 154.

In an embodiment of the present disclosure, the antisense oligonucleotide may be a sequence selected from the group consisting of SEQ ID NOs: 23, 75, 87, 112, and 144.

In an embodiment of the present disclosure, the antisense oligonucleotide may bind to at least 85 %, at least 90 %, at least 95 %, at least 97 %, at least 98 %, or at least 99 % of the nucleobases of the target sequence of SETD5 pre-mRNA by hybridization via Watson-Crick base pairings (A:T or G:C) or wobble base pairings (G:U, I:A, I:C, or I:U), thereby increasing SETD5 expression.

As used herein, the term "antisense oligonucleotide" refers to a nucleic acid sequence complementary to a target DNA or mRNA sequence, and is used interchangeably with "antisense oligomer" or "ASO" herein. Antisense oligonucleotides are generally designed to bind to a target RNA and modulate the expression or activity of the target at the transcriptional, translational, or splicing level. Such modulation may not only suppress the expression, but in some cases may also induce an increase in the expression. Antisense oligonucleotides are designed to hybridize with a gene, for example, SETD5, or its transcript under cellular conditions. Therefore, the oligonucleotides are designed to be sufficiently complementary to their targets, that is, to hybridize with sufficient specificity to their targets to produce a desired effect under cellular conditions.

As used herein, the term "5'-untranslated region (5'-UTR)" refers to a region of mRNA that is important for regulation of translation of the transcript. The 5'-UTR plays a key role in regulating translation efficiency, but does not itself encode a protein. For this specific feature, it is a promising target for increasing protein production without altering the coding sequence. The 5'-UTR begins at the transcription start site and ends at the nucleotides preceding the start codon (generally, AUG) of the coding region.

As used herein, the term "target region of the pre-mRNA of the *SETD5* gene" or "target region of *SETD5* pre-mRNA" refers to a region present in *SETD5* pre-mRNA which is targeted by an oligonucleotide to induce upregulation of SETD5 expression. The target region is located between exon 3 and exon 4 of *SETD5* pre-mRNA based on NM_001080517.3, and may be exon 3a or 3b, a part thereof, or a surrounding region including the same, which acts as a translation inhibitory region. An antisense oligonucleotide designed to bind thereto may act to induce splicing so that exons 3a and 3b are not included in the mRNA.

As used herein, the term "SETD5" refers to SET domain containing protein 5. SETD5 is a gene associated with SETD5 syndrome, a neurodevelopmental disorder characterized by intellectual disability and autism-like symptoms. SETD5 syndrome was first reported in 2014, and to date, no effective treatment has been identified. Polysome profiling and translation prediction models confirmed that a portion of the 5'-UTR region of the *SETD5* gene inhibits translation, which may serve as a suitable target for ASO-based regulation of *SETD5* translation.

As used herein, the term "splicing" refers to the process by which pre-mRNA transcribed from DNA is converted into mature mRNA, in which introns are removed from the pre-mRNA and exons are joined. The term "alternative splicing" refers to the production of mRNAs, that is, mRNA isoforms, with different structures or compositions depending on which introns or exons are removed or retained from a single pre-mRNA, and is used interchangeably herein with "selective splicing".

As used herein, the term "exon" refers to a portion of a gene that encodes a part of the final mature RNA from which introns have been removed by RNA splicing, and refers to the corresponding sequence in the DNA sequence of the gene and in the RNA transcript.

As used herein, the term "intron" refers to a nucleotide sequence within a gene that is removed by RNA splicing during maturation of the final RNA product, and refers to the corresponding to sequence in the DNA sequence of the gene and in the RNA transcript.

As used herein, the term "exon skipping" or "exon skip" refers to the production of mRNA from which a corresponding exon is omitted by the splicing of pre-mRNA. For example, when an antisense oligonucleotide targeting the corresponding exon binds thereto, the formation of a spliceosome complex mediating splicing is inhibited, and as a result, an mRNA that does not include the exon may be produced.

As used herein, the term "productive isoform" refers to an isoform of a final mRNA obtained by splicing of pre-mRNA, which does not include an exon acting as a translation inhibitory region, and means an mRNA that can be translated with higher translational activity compared to the case where the translation inhibitory region is present. As used herein, the term "non-productive isoform" refers to an mRNA isoform obtained by splicing of pre-mRNA, which includes a translation inhibitory region, and means an mRNA that cannot be translated into a final product, an mRNA that may be translated in a truncated form lacking a portion of the N-terminus compared to a full-length protein translated from SETD5 MANE (Matched Annotation from NCBI and EMBL-EBI) transcript, i.e., NM_001080517.3, or an mRNA that may be translated at reduced yield due to suppressed translational activity, or into a final product with weakened or impaired function.

As used herein, the term "translation inhibitory region" refers to a site or region composed of one or more nucleotides on the pre-mRNA, and means a site or region that, when included in the final mature mRNA, results in lower translational activity compared to the case where it is not included. The translation inhibitory region may be an exon. As used herein, the term "translation inhibitory region" is used interchangeably with "translation inhibitory factor" or "translation inhibitory element".

As used herein, the term "translational activity" refers to the activity of an mRNA being translated into a protein, and may be measured by the amount or level of protein or the number of ribosomes bound per mRNA. For translation, the greater the number of ribosomes bound per mRNA, the higher the translational activity, and the smaller the number of ribosomes bound per mRNA, the lower the translational activity may be. The translational activity may be analyzed by polysome profiling and translation prediction models.

As used herein, the term "complementarity" refers to the ability of nucleosides/nucleotides to form Watson-Crick base pairs. The Watson-Crick base pairs are guanine (G)-cytosine (C) and adenine (A)-thimine (T)/uracil (U). The oligonucleotide may include nucleosides having modified nucleobases, for example, 5-methylcytosine is often used instead of cytosine. Accordingly, the complementarity includes Watson-Crick base pairing between unmodified nucleobases and modified nucleobases.

As used herein, the term "Watson-Crick base pair" refers to a base pair between A and T (A:T) or between C and G (G:C), and the term "wobble base pair" refers to a base pair between G and U(G:U), I and A (I:A), I and C (I:C), or I and U (I:U).

As used herein, the term "% complementarity" refers to the proportion of complementary nucleotides capable of forming base pairs among nucleic acid molecules. The % complementarity is calculated by counting the number of aligned bases that form pairs between two sequences, for example, a reference sequence and a target sequence, dividing by the total number of nucleotides in the reference sequence, and multiplying by 100. In the comparison of two sequences, nucleobases/nucleotides that are not aligned, that is, those that do not form base pairs, are referred to as mismatches. The % complementarity of an antisense oligonucleotide to a target region is calculated by dividing the number of nucleotides in the antisense oligonucleotide that are capable of forming base pairs with nucleotides of the target region via Watson-Crick base pairing or wobble base pairing by the total length of the target region, that is, the total number of nucleotides constituting the target region, and multiplying by 100. The antisense oligonucleotide may have 80 % or more complementarity to the target region, for example, 85 %, 90 %, 91 %, 92 %, 93 %, 94 %, 95 %, 96 %, 97 %, 98 %, 99 %, or 100 % complementarity. The antisense oligonucleotide may include one, two, or three base mismatches relative to the target region, or may include one or more base mismatches as long as it has sufficient complementarity to effectively bind to the target region.

As used herein, the term "homology" refers to the proportion of positions at which the types of amino acids or in the case of bases, nucleotides match between polymers such as polypeptides or polynucleotides being compared, and may be used interchangeably with "identity" with respect to a sequence. Two sequences may be considered "homologous" to each other if the sequences are 80 % or more, 85 % or more, 90 %, 95 %, or 99 % or more identical. For example, the calculation of homology (%) between two polynucleotide sequences is performed by aligning the two sequences for optimal comparison, comparing the nucleotides at corresponding nucleotide positions, and determining that the two sequences are identical at a given position when the nucleotide at a position in the first sequence is the same as the nucleotide at the corresponding position in the second sequence. Sequence homology may be determined using softwares such as BLASTP, BLASTN, and FASTA.

In an embodiment of the present disclosure, the antisense oligonucleotide may bind to a target region of the SETD5 pre-mRNA and induce splicing to produce a productive isoform of SETD5 mRNA that does not include a region inhibiting translation of SETD5, thereby increasing the expression of SETD5.

Through polysome profiling and translation prediction models, it was confirmed by a luciferase assay that the expression of the protein increased when the 5'-UTR was constructed so that two exons (3a and 3b), which were identified to cause low translational activity, were not included in the mRNA. Therefore, it can be inferred that exons causing low translational activity contain a translation inhibitory region, and in the present disclosure, the generation of a productive isoform was increased by regulating splicing through binding of an antisense oligonucleotide to the corresponding region.

It is not necessarily required that the antisense oligonucleotide has 100 % sequence complementarity to the target region in order to exert intended effect through binding (hybridization) to the target region. The binding of the antisense oligonucleotide to the target region may lead to the formation of a complex in which the antisense oligonucleotide binds to the target region and prevents the target region from being spliced into mRNA, thereby allowing the generation of a productive isoform mRNA. Therefore, the antisense oligonucleotide and the target region only need to be complementary enough to induce the mRNA of a productive isoform. For example, the antisense oligonucleotide and the target region may have 80 % or more, 85 % or more, 90 % or more, 95 % or more, 98 % or more, 99 % or more, or 100 % complementarity.

The antisense oligonucleotide comprises 13 to 40 consecutive nucleotides, and at least 13 consecutive nucleotides bind to the target region through hybridization by forming Watson-Crick base pairs or wobble base pairs, to exert the intended effect, thereby increasing SETD5 expression. The antisense oligonucleotide may comprise 13 or more, more consecutive complementary bases to the target region, for example, 14, 15, 16, 17 or more consecutive complementary bases, and can exert the intended effect as long as it has sufficient complementarity to bind to the target region and induce splicing into an mRNA of a productive isoform in which the target region is skipped.

In an embodiment of the present disclosure, the antisense oligonucleotide may include one or more mismatches with the target nucleic acid region of the *SETD5* 5'-UTR. Despite the mismatches, hybridization of the antisense oligonucleotide to the target region may still be sufficient to induce selective splicing that leads to an increase in SETD5 expression. A reduction in binding affinity resulting from mismatches may advantageously be compensated by an increase in the number of nucleotides in the oligonucleotide and/or by an increase in the number of modified nucleosides capable of enhancing binding affinity to the target, for example, 2'-modified nucleosides, including LNA (locked nucleic acid), present within oligo nucleotide sequences.

In an embodiment of the present disclosure, the antisense oligonucleotide may comprise or consist of a sequence selected from the group consisting of SEQ ID NOs: 22 to 36, 38 to 40, 69 to 90, 110 to 112, 115, 142 to 145, 147, 152 to 154, 158, and 159, or a sequence having at least 80 %, 85 %, 90 %, 95 %, 97 %, 98 %, or 99 % homology thereto.

In an embodiment of the present disclosure, the antisense oligonucleotide may comprise or consist of a sequence selected from the group consisting of SEQ ID NOs: 23 to 28, 30, 32, 33, 70, 75, 77, 82, 87 to 89, 112, 142 to 144, 153, and 154, or a sequence having at least 80 %, 85 %, 90 %, 95 %, 97 %, 98 %, or 99 % homology thereto.

In an embodiment of the present disclosure, the antisense oligonucleotide may comprise or consist of a sequence selected from the group consisting of SEQ ID NOs: 23 to 28, 70, 75, 77, 87, 112, 143, 144, 153, and 154, or a sequence having at least 80%, 85%, 90%, 95%, 97%, 98%, or 99% homology thereto.

In an embodiment of the present disclosure, the antisense oligonucleotide may comprise or consist of a sequence selected from the group consisting of SEQ ID NOs: 23, 75, 87, 112, and 144, or a sequence having at least 80 %, 85 %, 90 %, 95 %, 97 %, 98 %, or 99 % homology thereto.

The antisense oligonucleotide was designed to have complementarity to the target region and to induce splicing in a manner that increases the generation of a productive isoform mRNA, and was selected by confirming its activity. If the antisense oligonucleotide binds to the target region or its adjacent region including the target region, and induces splicing such that the target region, i.e., the region having translational inhibitory activity, is not included in the mRNA, the intended effect can be considered achieved. Therefore, an antisense oligonucleotide or an antisense oligonucleotide having at least 80 % homology thereto, for example, at least 85 %, 90 %, 95 %, 98 %, 99 %, or 100 % homology, may effectively function.

A plurality of antisense oligonucleotides that bind to the same target region and adjacent regions thereof were designed, and their binding to the target region and the resulting effects on splicing were verified, thereby confirming that specific antisense oligonucleotide sequences and antisense oligonucleotides having at least 80 % sequence identity thereto exhibited the same or similar effects.

In an embodiment of the present disclosure, the antisense oligonucleotide may comprise one or more modifications selected from chemically modified sugar moieties, chemically modified bases, and chemically modified internucleoside linkages.

In an embodiment of the present disclosure, the chemically modified sugar moiety may be selected from sugar moieties modified with 2'-O-methyl, 2'-methoxyethoxy, 2'-O-methoxyethyl (2'-MOE), 2'-dimethylaminoethoxy, 2'-dimethylaminoethoxyethoxy, 2'-fluoro, 2'-acetamide, morpholino, LNA (locked nucleic acid), and c-ET (S-constrained-ethyl),
the modified base may be selected from the group consisting of isocytosine, pseudoisocytosine, 5-methylcytosine, 5-fluorocytosine, 5-thiazolylcytosine, 5-propynylcytosine, 5-propynyluracil, 5-bromouracil 5-thiazolyluracil, 2-thiouracil, 2'-thiothymine, inosine, diaminopurine, 6-aminopurine, 2-aminopurine, 2,6-diaminopurine, and 2-chloro-6-aminopurine, and/or
the chemically modified internucleoside linkage may be a phosphorothioate bond or a phosphorodiamidate bond.

**In** an embodiment of the present disclosure, the antisense oligonucleotide may comprise one or more modifications selected from chemically modified sugar moieties, chemically modified bases, and chemically modified internucleoside linkages, wherein
wherein the chemically modified sugar moiety comprises at least one sugar moiety modified with 2'-O-methyl, 2'-methoxyethoxy, 2'-O-methoxyethyl (2'-MOE), 2'-dimethylaminoethoxy, 2'-dimethylaminoethoxyethoxy, 2'-fluoro, 2'-acetamide, morpholino, locked nucleic acid (LNA), or S-constrained-ethyl (c-ET),
the modified base comprises at least one of 5-methylcytosine, 5-fluorocytosine, and 2-aminopurine base, and
the chemically modified internucleoside linkage comprises a phosphorothioate linkage.

In an embodiment of the present disclosure, the antisense oligonucleotide consists of nucleosides having a 2'-MOE modification, the internucleoside linkages are phosphorothioate, and all cytosine (C) bases are 5'-methylcytosines for all cytosine (C) bases.

In an embodiment of the present disclosure, the antisense oligonucleotide may comprise nucleotides in which all nucleosides are 2'-MOE modified, the internucleoside linkages are phosphorothioate linkages, and all cytosine (C) bases are 5'-methylcytosines.

In an embodiment of the present disclosure, the antisense oligonucleotide comprises 13 to 40 consecutive nucleotides, and binds to a target region of SETD5 pre-mRNA by hybridization via at least 13 consecutive Watson-Crick base pairings (A:T or G:C) or wobble base pairings (G:U, I:A, I:C, or I:U); or 80 % or more of nucleobases of the target region on SETD5 pre-mRNA by hybridization via Watson-Crick base pairings (A:T or G:C) or wobble base pairings (G:U, I:A, I:C, or I:U), to increase SETD5 expression,
wherein the target region comprises a sequence selected from the group consisting of SEQ ID NOs: 1 to 15, 17 to 19, 47 to 68, 95 to 97, 100, 121 to 124, 126, 131 to 133, 137, and 138,
the antisense oligonucleotide comprises at least one modification selected from a chemically modified sugar moiety, a chemically modified base, and a chemically modified internucleoside linkage,
wherein the chemically modified sugar moiety comprises at least one sugar moiety modified with 2'-O-methyl, 2'-methoxyethoxy, 2'-O-methoxyethyl (2'-MOE), 2'-dimethylaminoethoxy, 2'-dimethylaminoethoxyethoxy, 2'-fluoro, 2'-acetamide, morpholino, locked nucleic acid (LNA), or S-constrained-ethyl (c-ET),
the modified base comprises at least one of 5-methylcytosine, 5-fluorocytosine, and 2-aminopurine base, and
the chemically modified internucleoside linkage comprises a phosphorothioate linkage.

In an embodiment of the present disclosure, the antisense oligonucleotide may comprise or consist of a sequence selected from the group consisting of SEQ ID NOs: 22 to 36, 38 to 40, 69 to 90, 110 to 112, 115, 142 to 145, 147, 152 to 154, 158, and 159, wherein all nucleosides may be nucleosides with 2'-MOE modification, the internucleoside linkages may be phosphorothioate linkages, and all C bases may be 5'-methylcytosines.

In an embodiment of the present disclosure, the antisense oligonucleotide may comprise or consist of a sequence selected from the group consisting of SEQ ID NOs: 23 to 28, 30, 32, 33, 70, 75, 77, 82, 87 to 89, 112, 142 to 144, 153, and 154, wherein all nucleosides are nucleosides with 2'-MOE modification, the internucleoside linkages are phosphorothioate, and all C bases are 5'-methyl-cytosines.

In an embodiment of the present disclosure, the antisense oligonucleotide may comprise or consist of a sequence selected from the group consisting of SEQ ID NOs: 23 to 28, 70, 75, 77, 87, 112, 143, 144, 153, and 154, wherein all nucleosides have a 2'-MOE modification, the internucleoside linkages are phosphorothioate, and all C bases are 5'-methylcytosines.

In an embodiment of the present disclosure, the antisense oligonucleotide may comprise or consist of a sequence selected from the group consisting of SEQ ID NOs: 23, 75, 87, 112, 144, 153, and 154, wherein all nucleosides have a 2'-MOE modification, the internucleoside linkages are phosphorothioate, and all C bases are 5'-methylcytosines.

In an embodiment of the present disclosure, the antisense oligonucleotide may have a structure of one of Formulae (I) to (VII):

G*G*C*T*T*C*C*A*C*T*T*C*C*T*T*T*C*T*G*T Formula (I);

G*G*G*C*T*T*C*C*A*C*T*T*C*C*T*T*T*C*T*G*T Formula (II);

A*C*A*T*C*C*T*G*G*T*G*T*A*G*T*G*C*C*A*C Formula (III);

A*T*G*A*T*C*A*A*A*G*T*G*C*C*T*T*C*T*G*C Formula (IV);

T*G*A*G*A*T*G*A*T*C*A*A*A*G*T*G*C*C*T*T*C Formula (V);

A*T*G*A*T*C*A*A*A*G*T*G*C*C*T*T*C*T*G Formula (VI);

and

A*T*G*A*T*C*A*A*A*G*T*G*C*C*T*T*C*T Formula (VII).

wherein A is 2'MOE-A, C is 2'MOE-5'-methyl-C, G is 2'MOE-G, T is 2'MOE-T, * is phosphorothioate (PS), and 2'MOE is 2'-O-methoxyethyl.

In an embodiment of the present disclosure, the antisense oligonucleotide may have a structure of Formulae (I) and (IV):

G*G*C*T*T*C*C*A*C*T*T*C*C*T*T*T*C*T*G*T Formula (I);

and

A*T*G*A*T*C*A*A*A*G*T*G*C*C*T*T*C*T*G*C Formula (IV);

wherein A is 2'MOE-A, C is 2'MOE-5'-methyl-C, G is 2'MOE-G, T is 2'MOE-T, * is phosphorothioate (PS), and 2'MOE is 2'-O-methoxyethyl.

As used herein, the term "modified oligonucleotide" refers to an oligonucleotide comprising one or more sugar-modified nucleosides and/or modified internucleoside linkages.

As used herein, the term "chemical modification" refers to the introduction of chemically modified nucleosides or internucleoside linkages into an antisense oligonucleotide, such as an antisense oligonucleotide sequence, to confer nuclease resistance and/or to increase binding affinity to the target nucleic acid.

As used herein, the term "modified nucleoside" refers to a nucleoside that is modified relative to a corresponding DNA or RNA nucleoside by the introduction of one or more modifications in the sugar moiety or (nucleo)base moiety.

As used herein, the term "internucleoside linkage" or "internucleoside bond" refers to a linkage or bond connecting two nucleosides via a covalent bond, for example, a phosphodiester (PO) linkage, but not limited thereto. For natural oligonucleotides, the internucleoside linkages may include phosphate groups that form phosphodiester linkages between adjacent nucleosides. A modified internucleoside linkage increases the nuclease resistance of an oligonucleotide as compared to a PO linkage. Modified internucleoside linkages are particularly useful for stabilizing oligonucleotides for in vivo use. The modified internucleoside linkage or linking group may include a phosphorothioate linkage or a phosphorodiamidate linkage, but is not limited thereto.

As used herein, the term "nucleobase" refers to the purine (e.g., adenine and guanine) and pyrimidine (e.g., uracil, thymine, and cytosine) residues present in nucleosides and nucleotides that form hydrogen bonds during hybridization of nucleic acids. As used herein, the term "nucleobase" includes modified nucleobases that differ from natural nucleobases but remain functional during hybridization of nucleic acids. In this regard, the term "nucleobase" includes not only natural nucleobases, such as adenine, guanine, cytosine, thymidine, uracil, xanthine, and hypoxanthine, but also non-natural variants thereof.

Another aspect of the present disclosure provides a pharmaceutical composition for treating a disease associated with abnormal level and/or activity of SETD5, comprising the aforementioned antisense oligonucleotide.

In an embodiment of the present disclosure, the disease may be SETD5 syndrome.

Another aspect of the present disclosure provides a pharmaceutical composition for treating a disease for which a therapeutic effect has been confirmed by upregulation of SETD5 expression, comprising a virus or vector that expresses the aforementioned antisense oligonucleotide in cells.

In an embodiment of the present disclosure, the disease may be SETD5 syndrome.

In an embodiment of the present disclosure, the antisense oligonucleotide may comprise a sequence selected from the group consisting of SEQ ID NOs: 22 to 36, 38 to 40, 69 to 90, 110 to 112, 115, 142 to 145, 147, 152 to 154, 158, and 159 or a sequence having at least 80 % homology thereto.

In an embodiment of the present disclosure, the antisense oligonucleotide may comprise a sequence selected from the group consisting of SEQ ID NOs: 23 to 28, 30, 32, 33, 70, 75, 77, 82, 87 to 89, 112, 142 to 144, 153, and 154; or a sequence having at least 80% homology thereto.

In an embodiment of the present disclosure, the antisense oligonucleotide may comprise a sequence selected from the group consisting of SEQ ID NOs: 23 to 28, 70, 75, 77, 87, 112, 143, 144, 153, and 154 or a sequence having at least 80% homology thereto.

In an embodiment of the present disclosure, the antisense oligonucleotide may comprise a sequence selected from the group consisting of SEQ ID NOs: 23, 75, 87, 112, and 144, or a sequence having at least 80% homology thereto.

In an embodiment of the present disclosure, the antisense oligonucleotide may comprise a sequence selected from the group consisting of SEQ ID NOs: 23, 75, 87, 112, 144, 153, and 154, or a sequence having at least 80% homology thereto.

In an embodiment of the present disclosure, the antisense oligonucleotide may comprise nucleotides in which all nucleosides are 2'-MOE modified, the internucleoside linkages are phosphorothioate linkages, and all cytosine (C) bases are 5'-methylcytosines.

In an embodiment of the present disclosure, the antisense oligonucleotide may be a combination of antisense oligonucleotides having different sequences.

In an embodiment of the present disclosure, the antisense oligonucleotide may have a structure of one of Formulae (I) to (VII):

G*G*C*T*T*C*C*A*C*T*T*C*C*T*T*T*C*T*G*T Formula (I);

G*G*G*C*T*T*C*C*A*C*T*T*C*C*T*T*T*C*T*G*T Formula (II);

A*C*A*T*C*C*T*G*G*T*G*T*A*G*T*G*C*C*A*C Formula (III);

A*T*G*A*T*C*A*A*A*G*T*G*C*C*T*T*C*T*G*C Formula (IV);

T*G*A*G*A*T*G*A*T*C*A*A*A*G*T*G*C*C*T*T*C Formula (V);

A*T*G*A*T*C*A*A*A*G*T*G*C*C*T*T*C*T*G Formula (VI);

and

A*T*G*A*T*C*A*A*A*G*T*G*C*C*T*T*C*T Formula (VII).

wherein A is 2'MOE-A, C is 2'MOE-5'-methyl-C, G is 2'MOE-G, T is 2'MOE-T, * is phosphorothioate (PS), and 2'MOE is 2'-O-methoxyethyl.

In an embodiment of the present disclosure, the pharmaceutical composition may further comprise pharmaceutically acceptable excipients, additives, or the like.

In an embodiment of the present disclosure, the pharmaceutical composition may further comprise other therapeutic agents for treating a disease associated with abnormal level and/or activity of SETD5. The antisense oligonucleotide and the other therapeutic agents may be administered simultaneously or separately.

The pharmaceutical composition according to the present disclosure is administered in a therapeutically effective amount, that is, an amount sufficient to produce a desired therapeutic effect. The therapeutically effective amount is preferably be an amount sufficient to treat a disease associated with abnormal level and/or activity of SETD5, and may be determined in consideration of specific factors, including the body weight, gender, and age of the subject to be treated, the particular composition administered, the active ingredient(s) in the composition, the timing and route of administration, the overall health of the subject, and any other concurrently administered drugs. The pharmaceutical composition according to the present disclosure may be administered, for example, by oral administration or by parenteral administration, such as intrathecal injection, subcutaneous injection, intravenous injection or infusion, intraarterial injection or infusion, intramuscular injection, topical administration, or administration to a target organ. The antisense oligonucleotide according to the present disclosure may be administered intrathecally, at a dose in the range of 1 mg to 100 mg, 1 mg to 60 mg, 1 mg to 40 mg, 1 mg to 20 mg, or 5 mg to 50 mg, but is not limited thereto.

As used herein, the term "SETD5 syndrome" refers to a rare disorder caused by a mutation in the *SETD5* gene located on chromosome 3, which generally occurs when the level or activity of functional SETD5 is insufficient or reduced due to a mutation in the *SETD5* gene. Therefore, symptoms can be treated or ameliorated by increasing SETD5 expression to elevate the level/activity of SETD5 to a normal range.

As used herein, the term "disease associated with abnormal level or activity of SETD5" refers to a disease that occurs when the level or activity of functional SETD5 is lower or insufficient compared to normal level or activity, and may, for example, include SETD5 syndrome.

Another aspect of the present disclosure provides a method for treating a disease associated with abnormal level or activity of SETD5, the method comprising administering a therapeutically effective amount of the aforementioned antisense oligonucleotide to a subject.

In an embodiment of the present disclosure, the disease is SETD5 syndrome.

In an embodiment of the present disclosure, the antisense oligonucleotide comprises 13 to 40 consecutive nucleotides, and binds to a target region of SETD5 pre-mRNA by hybridization via at least 13 consecutive Watson-Crick base pairings (A:T or G:C) or wobble base pairings (G:U, I:A, I:C, or I:U); or 80 % or more of nucleobases of the target region on SETD5 pre-mRNA by hybridization via Watson-Crick base pairings (A:T or G:C) or wobble base pairings (G:U, I:A, I:C, or I:U), to increase SETD5 expression,
wherein the target region comprises a sequence selected from the group consisting of SEQ ID NOs: 1 to 15, 17 to 19, 47 to 68, 95 to 97, 100, 121 to 124, 126, 131 to 133, 137, and 138.

As used herein, the term "therapeutic effective amount" refers to an amount of an active ingredient sufficient to achieve a desired therapeutic effect, and for example, may refer to an amount of an antisense oligonucleotide that can lead to alleviation, mitigation, amelioration, or treatment of SETD5 syndrome or symptoms thereof.

In an embodiment of the present disclosure, the antisense oligonucleotide of the present disclosure may be administered via a non-oral route, including intravenous, intraarterial, subcutaneous, intraperitoneal, or intramuscular injection or infusion, or intrathecal or intracranial administration, for example, intracerebral or intraventricular administration.

### Advantageous Effects of Invention

The antisense oligonucleotide according to an embodiment of the present disclosure has high specificity for a target region of the *SETD5* gene, inhibits the generation of nonproductive isoforms and increases the generation of productive isoforms, thereby inducing an increase in normal SETD5 protein expression, and can thus be effectively used for the treatment of diseases associated with abnormal level and/or activity of SETD5, for example, SETD5 syndrome.

### Brief Description of Drawings

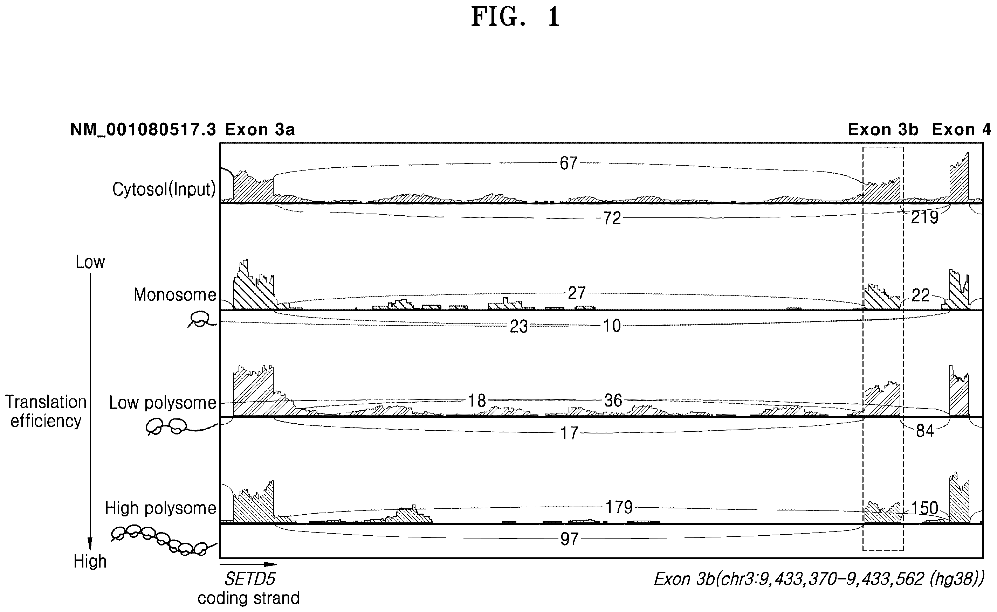

FIG. 1 shows the analysis of human neuron polysome profiling data for the identification of a portion corresponding to exon 3b in a target region of *SETD5* 5'-untranslated region (5'-UTR) according to an embodiment of the present disclosure. From polysome profiling data (GEO accession number: GSE100007) obtained by separating mRNAs present in human neuronal cells cultured for 50 days according to the number of bound ribosomes (translation efficiency), and performing RNA-seq on each fraction, it was found that when exon 3b, located between exon 3a and exon 4 within the 5'-UTR region of SETD5 pre-mRNA (NM_001080517.3), is included through splicing, translation efficiency is reduced, thereby identifying the presence of translation-inhibitory elements within this region. The uppermost track shows the RNA-seq results for the input used in the cytoplasmic polysome profiling experiment.

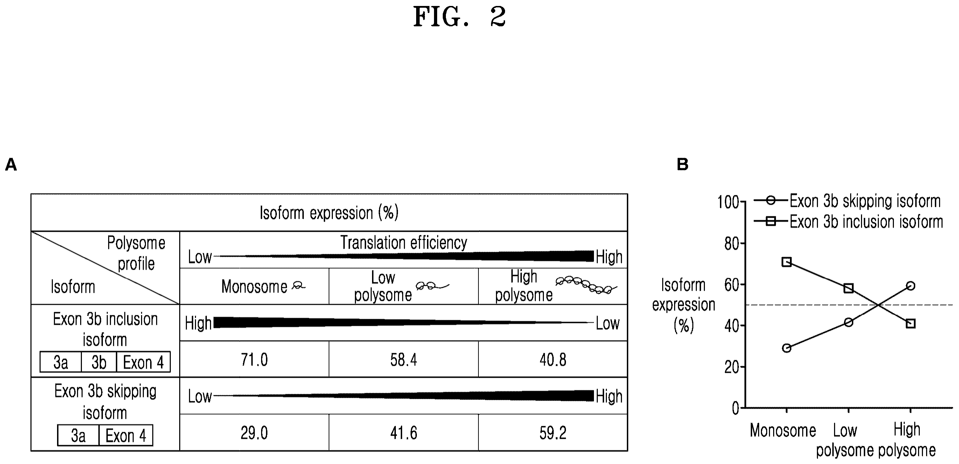

FIG. 2 shows the results of an analysis of human neuron polysome profiling data (GEO accession number: GSE100007) targeting exon 3b, performed to validate a target region within the *SETD5* 5'-UTR according to an embodiment of the present disclosure. Panel A shows the quantification of relative expression levels of isoforms including or excluding exon 3b in monosome, low polysome, and high polysome fractions, which were separated based on translation activity corresponding to the number of ribosomes. Panel B shows that, from the monosome fraction to the high polysome fraction, the proportion of isoforms in which exon 3b is skipped (skipping isoform, solid line with open circles) increases, whereas the proportion of isoforms in which exon 3b is included (inclusion isoform, solid line with open squares) decreases. This suggests that isoforms including exon 3b are predominantly observed in fractions with low translation efficiency, whereas isoforms in which exon 3b is skipped are mainly found in fractions with relative high translation efficiency.

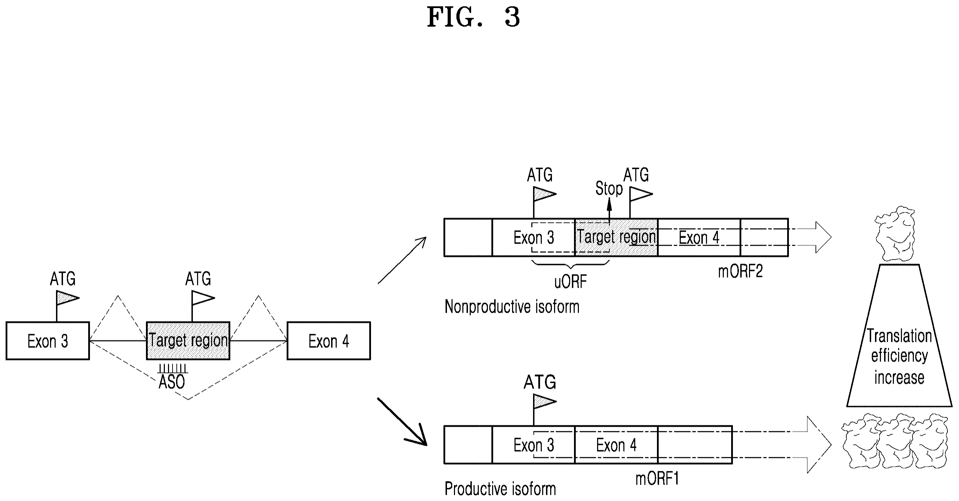

FIG. 3 is a schematic diagram showing a strategy in which an antisense oligonucleotide (ASO) targeting a region within 5'-UTR comprising a translation inhibitory element is used to selectively modulate splicing. This approach reduces the generation of nonproductive isoforms that include the exon harboring a translation inhibitory element, while simultaneously increasing the generation of productive isoforms that exclude the exon, thereby enhancing translation efficiency. For nonproductive isoforms containing the target region between exon 3 and exon 4, translation via main open reading frame (mORF) 2 can produce a protein that is shorter than the full-length protein encoded by the MANE transcript but retains partial functionality. However, the presence of an upstream open reading frame (uORF) starting in exon 3 impedes translation, resulting in low translation efficiency from mORF2. Conversely, isoforms from which the target region is excluded are classified as productive isoforms. In this case, the full-length protein is produced via mORF1, and the aforementioned uORF does not impede translation, resulting in high translation efficiency.

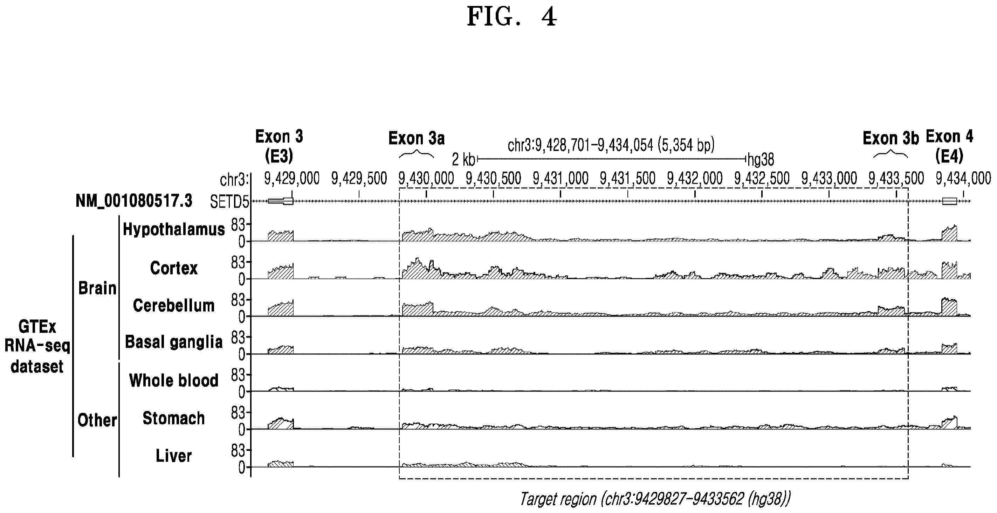

FIG. 4 shows the expression levels of the target region within the *SETD5* 5'-UTR in each human tissue according to an embodiment of the present disclosure. Using the GTEx RNA-seq dataset, the inclusion levels of the target region (hg38, chr3:9,429,827-9,433,562) of the SETD5 gene were determined after splicing in each human tissue. Compared to other tissues, the level of mRNA containing the target region within the *SETD5* gene was higher in brain tissues. The exon numbers are indicated based on the SETD5 MANE transcript NM_001080517.3.

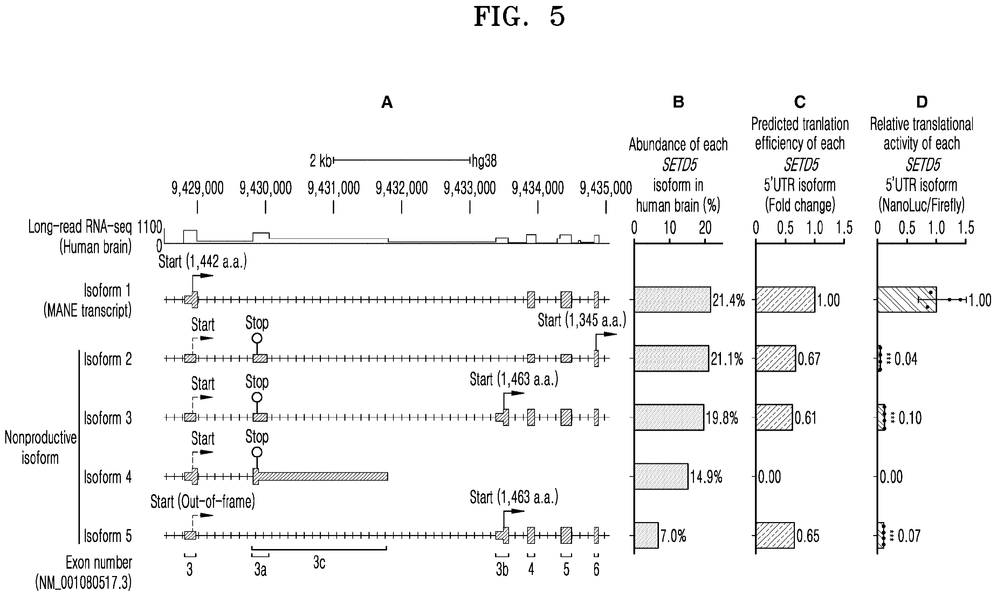

FIG. 5 shows RefSeq annotated isoforms in the 5'-UTR of the human *SETD5* gene. In Isoforms 1 to 3 and 5, the start codon positions of the main open reading frame (mORF) are indicated by solid arrows, and the start and stop codon positions of the upstream open reading frame (uORF) are indicated by dashed arrows. The graph at the top of panel A and the graphs in panel B show results obtained by analyzing a human brain long-read RNA-seq dataset (PRJDB15555), illustrating the abundance of each SETD5 isoform in the region encompassing the target site within the *SETD5* 5'-UTR and its surrounding sequences, according to one embodiment of the present disclosure. Panel C shows the results of predicting and comparing translational activity of each SETD5 transcript registered in the human RefSeq annotation by using the Framepool model (PMID: 33970899), which predicts the average number of ribosomes bound per mRNA based on the 5'-UTR sequence of a given transcript. The model predicted that translational activity varies depending on whether a specific exon within the 5'-UTR is included in the mature mRNA. Using this approach, it was predicted that, when exon 3a or exon 3b or both exon 3a and exon 3b of *SETD5* are skipped through splicing, the translational activity of the *SETD5* gene increases, leading to the production of the full-length protein. Based on this analysis, exons 3a and 3b were identified as target exons. Isoform 4 is a nonproductive isoform due to early transcription termination. However, because exon 3c of isoform 4 shares the splicing acceptor site with exon 3a, treatment with an ASO designed to induce skipping of exon 3a is also expected to cause skipping of exon 3c in isoform 4. This prevents early transcription termination of isoform 4, allowing transcription to proceed and resulting in the production of the productive isoform, isoform 1. Panel D shows the results of measuring the translational activity of the four 5'-UTR isoforms of the *SETD5* gene, depending on whether the target region within the 5'-UTR of SETD5, according to an embodiment of the present disclosure, is included. In panel D, the translational activity of each isoform is shown relative to isoform 1. The data is based on four biological replicates, and for each biological replicate, the value represents the geometric mean of three technical replicate measurements. Statistical analysis was performed using an unpaired two-tailed equal variance t-test to assess difference between geometric means of isoform 1 and other isoforms. Error bars represent the 95 % confidence interval of the geometric mean. ***, P < 0.001 (isoform 2, P = 0.0003; isoform 3, P = 0.0004; isoform 5, P = 0.0003).

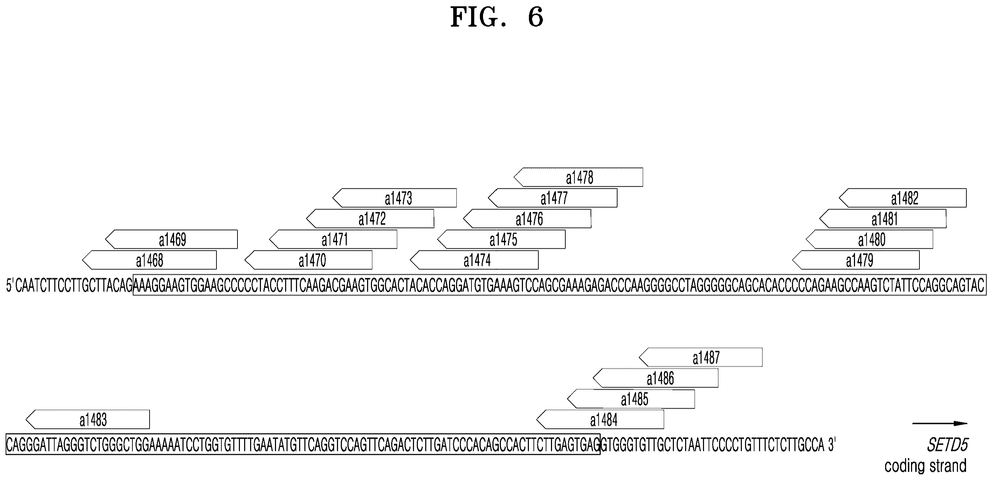

FIG. 6 shows exon 3a (225 bp; hg38, chr3:9,429,827-9,430,051), one of the target regions within the *SETD5* 5'-UTR according to an embodiment of the present disclosure, and the positions of 20 antisense oligonucleotides designed to target exon 3a and its surrounding sequences. The sequence shown in FIG. 6 corresponds to chr3:9,429,808-9,430,087 on the hg38 reference genome and is presented in the plus strand orientation. The boxed region corresponds to exon 3a, and the designed antisense oligonucleotides are indicated by triangular-headed boxes.

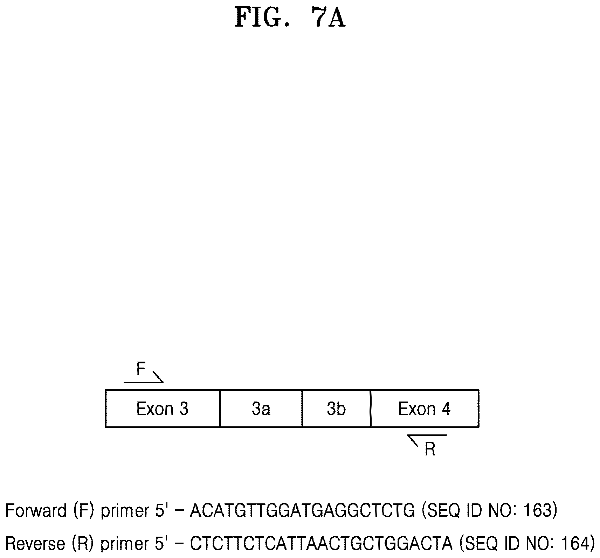

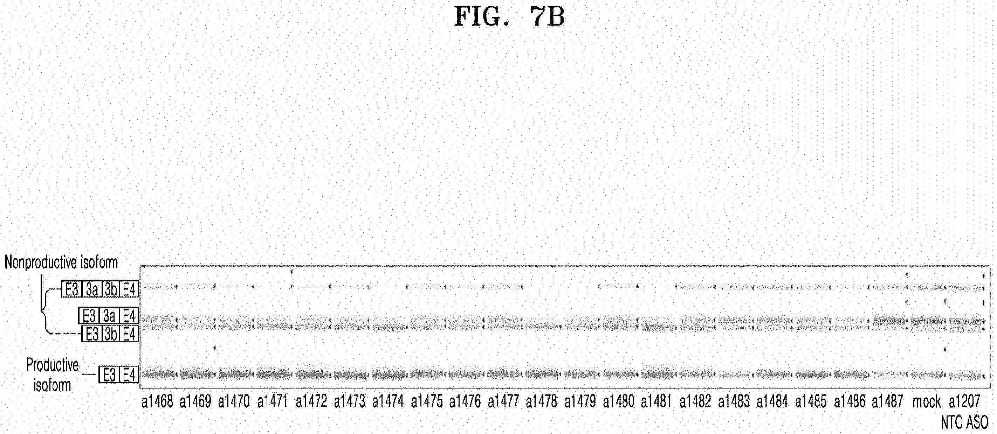

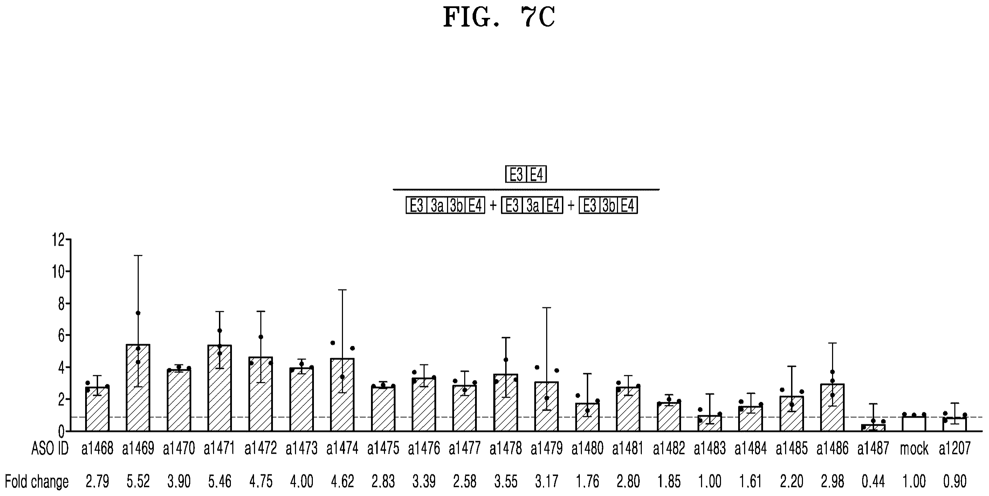

FIGS. 7A, 7B, and 7C show the expression of the target region within the *SETD5* 5'-UTR and the results of antisense oligonucleotide (ASO) screening, according to an embodiment of the present disclosure. FIG. 7A shows the positions and sequences of primers designed for RT-PCR, and FIGS. 7B and 7C show the results of screening ASOs designed for exon 3a, one of the target regions, and its surrounding sequences. FIG. 7B shows the RT-PCR results, and FIG. 7C presents the ratio of productive isoforms to nonproductive isoforms derived from the RT-PCR results, normalized to the mock control. The fold change relative to the mock is indicated below each ASO ID. Error bars represent the 95 % confidence interval of the geometric mean, and each point on the graph corresponds to a biological replicate (n=3). Mock refers to a control group treated under the same conditions with ultra pure water (UPW) instead of ASO.

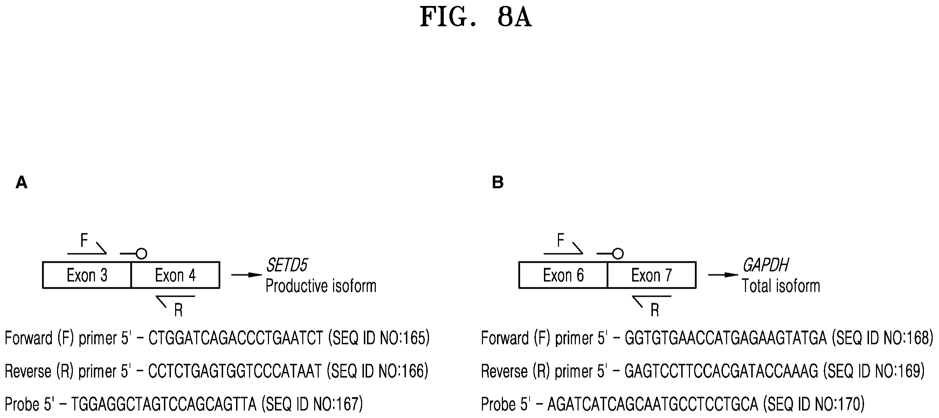

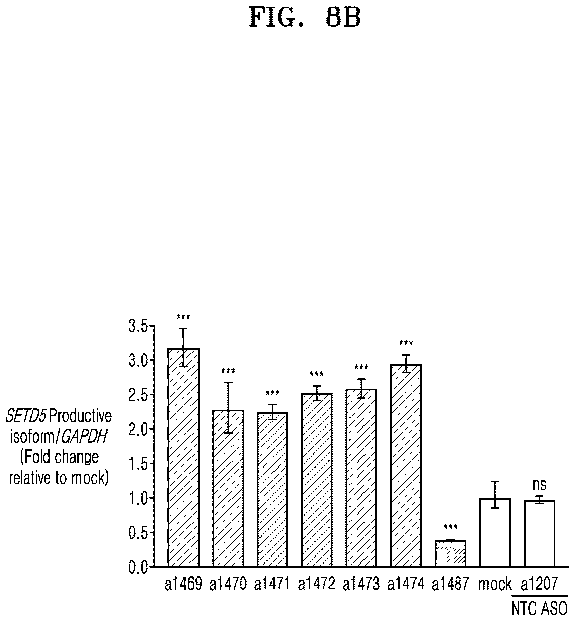

FIGS. 8A and 8B show the primers and probes used for RT-qPCR analysis of changes in the expression of the productive SETD5 isoform in U2OS cells treated with candidate ASOs designed to target a region within the *SETD5* 5'-UTR, according to an embodiment of the present disclosure. In FIG. 8A, panel A shows the primers and probe used for productive SETD5 isoform-specific RT-qPCR, designed based on the RefSeq transcript NM_001080517.3, while panel B shows the primers and probe used for GAPDH isoform-non-specific RT-qPCR, designed based on the RefSeq transcript NM_002046.7. FIG. 8B shows changes in the expression of productive SETD5 isoform in U2OS cells treated with candidate ASOs designed to target a region within the *SETD5* 5'-UTR, according to an embodiment of the present disclosure. In the RT-PCR analysis, RT-qPCR was performed to measure the expression of productive *SETD5* isoforms using six candidate ASOs that showed at least a threefold splicing effect compared to mock, ASO a1487 which showed a reduced effect compared to mock, and the control ASO a1207, with GAPDH used as an internal control. Unpaired two-tailed equal variance t-tests were performed to assess differences in the geometric means between mock and each treatment condition, and error bars represent the 95 % confidence interval of the geometric mean. ***, P < 1E-3 (a1469, P = 1E-5; a1470, P = 2E-4; a1471, P = 2E-5; a1472, P = 7E-6; a1473, P = 8E-6; a1474, P = 3E-6; a1487, P = 2E-4; a1207, P = 0.55, ns (not significant)).

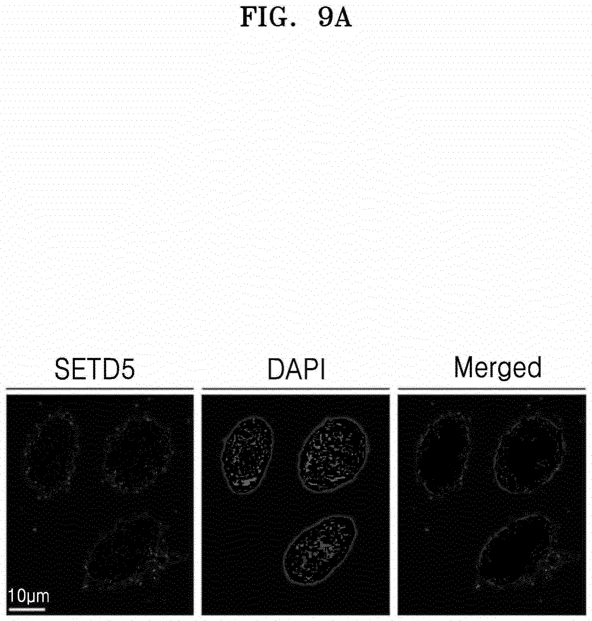

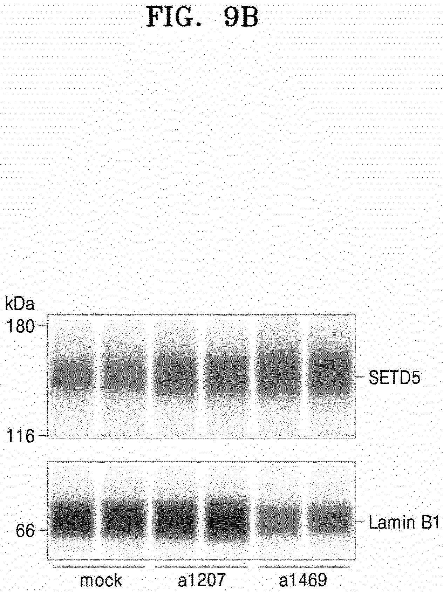

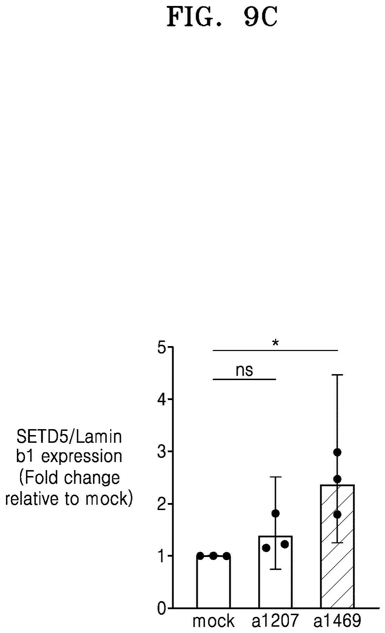

FIGS. 9A to 9C show changes in the expression of the SETD5 protein in the U2OS cells following treatment with ASO a1469 according to an embodiment of the present disclosure. FIG. 9A shows that the SETD5 protein is localized to the nucleus as determined by immunofluorescence analysis. SETD5 was stained green, the nucleus was stained with DAPI in blue, and the scale bar represents 10 µm. FIG. 9B shows the results of a capillary Western blot analysis of nuclear proteins isolated from U2OS cells treated with PBS (mock), a1469 (200 nM, candidate ASO), or a1207 (200 nM, control ASO), and FIG. 9C shows a graph quantifying the capillary Western blot results of FIG. 9B. Each point represents an independent biological replicate (n=3; mean of two technical replicate measurements), and the data are presented as the geometric mean ± 95 % confidence interval. An unpaired two-tailed equal variance t-test was performed to assess the difference in geometric means between the mock group and each treatment group. The a1469-treated group showed a significant difference compared with the mock group (*, P < 0.05; a1469, P = 0.0146), whereas the a1207-treated group showed no significant difference from the mock group (P = 0.1281, ns).

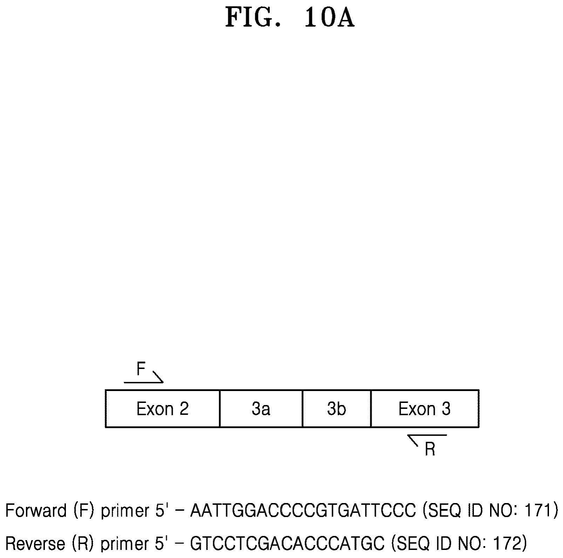

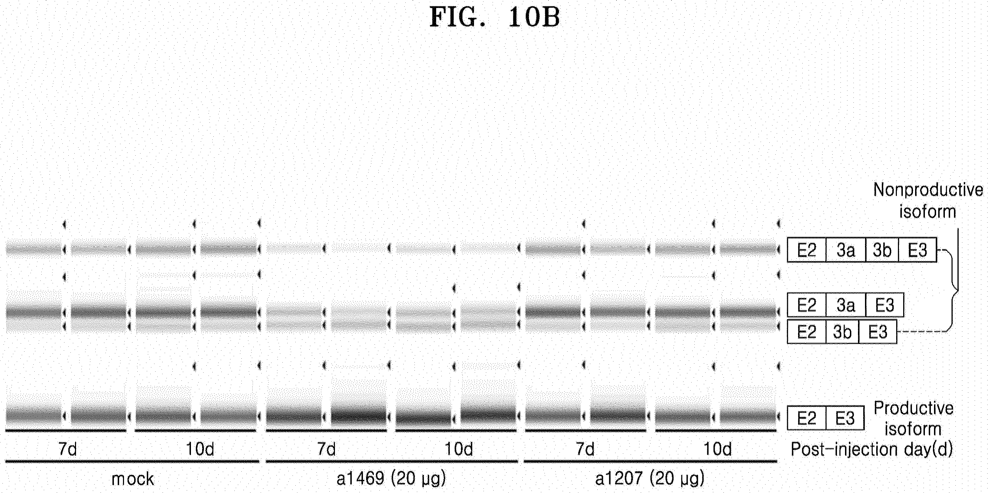

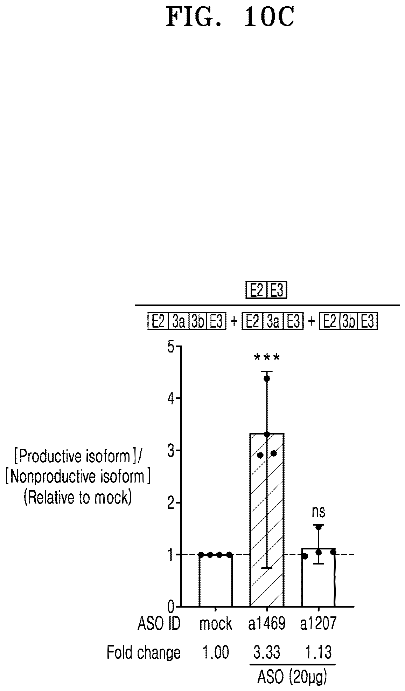

FIGS. 10A to 10C show the effect of treatment with a1469 according to an embodiment of the present disclosure on the regulation of SETD5 splicing in the mouse brain. FIG. 10A shows the positions and sequences of primers for mouse RT-PCR designed to examine the relative expression levels of isoforms associated with the target region within the *SETD5* 5'-UTR. FIG. 10B shows the results of RT-PCR analysis measuring changes in the expression of productive and nonproductive isoforms in RNA extracted from the cerebral cortex 7 or 10 days after intracerebroventricular (ICV) injection (left ventricle, 2 µL) of a1469 (20 µg) or control ASO a1207 (20 µg) into two-day-old C57BL/6N mice. FIG. 10C shows the results comparing the relative ratio of the productive isoform to the nonproductive isoforms with respect to the mock group, based on the RT-PCR results of FIG. 10B. Error bars represent the 95 % confidence interval of the geometric mean. Statistical analysis was performed using an unpaired two-tailed equal variance t-test on the geometric means of the mock group and each treatment group. As a result of the analysis, the a1469-treated group showed a significant difference compared with the mock group (***, P < 1E-3; a1469, P = 0.0004), whereas the a1207-treated group showed no significant difference compared with the mock group (P = 0.2795, ns).

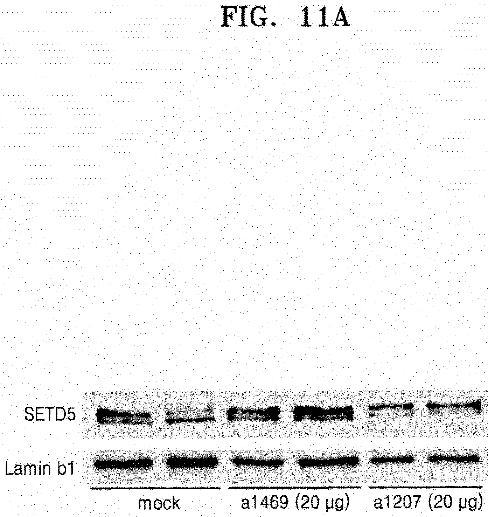

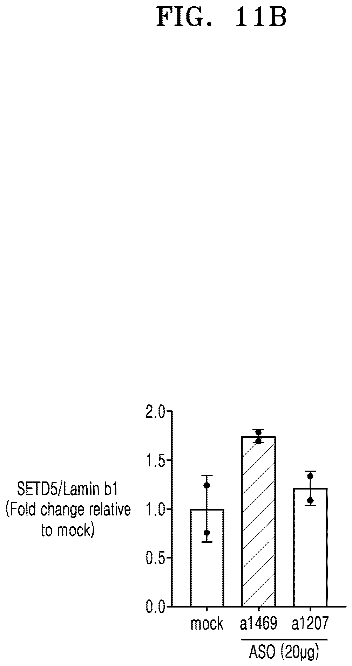

FIGS. 11A and 11B show changes in the expression of the SETD5 protein in the mouse brain following treatment with a1469 according to an embodiment of the present disclosure. FIG. 11A shows the results of a Western blot performed using nuclear protein extracted from the cerebral cortex following ICV injection (left ventricle, 2 µl) of a1469 (20 µg) or control ASO a1207(20 µg) into two-day-old C57BL/6N mice, and FIG. 11B is a graph showing the fold change in SETD5 protein expression relative to Lamin B1, compared to the mock group, based on the quantification of the Western blot results shown in FIG. 11A.

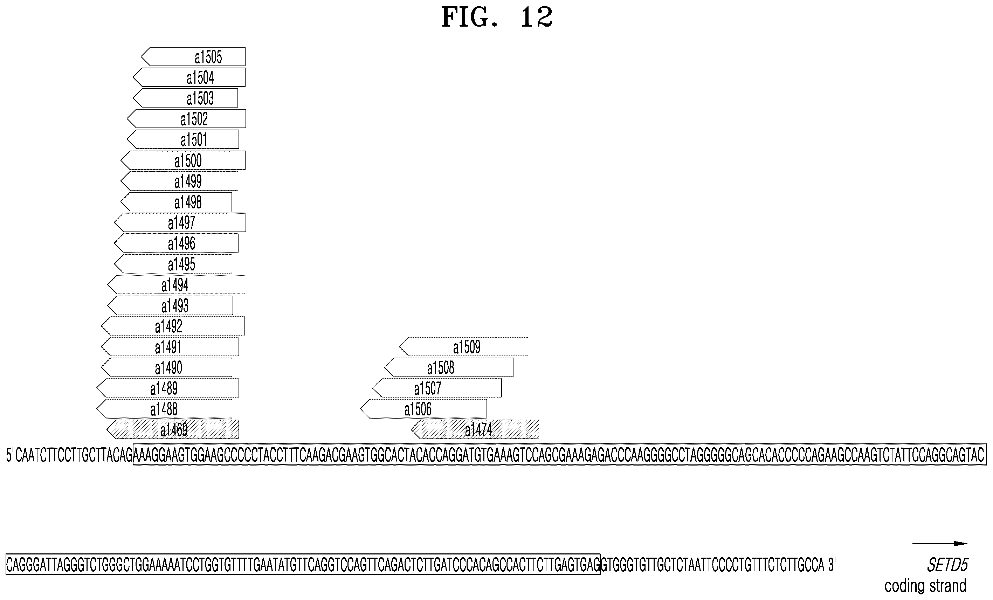

FIG. 12 shows the optimization design of ASOs for the target region within the *SETD5* 5'-UTR according to an embodiment of the present disclosure. A total of 22 additional ASOs (unshaded) were designed around the sequences of a1469 and a1474 (dot-shaded) which showed the highest efficacy in the primary screening. The target region, which includes the target exon (exon 3a) and its flanking sequences, corresponds to chr3:9,429,808-9,430,087 on the hg38 reference genome, and is shown in the direction of the plus strand of the genome, which corresponds to the coding strand of SETD5. The target exon region (exon 3a; hg38, chr3:chr3:9,429,827-9,430,051, 225 bp) of the designed ASOs is indicated by shading.

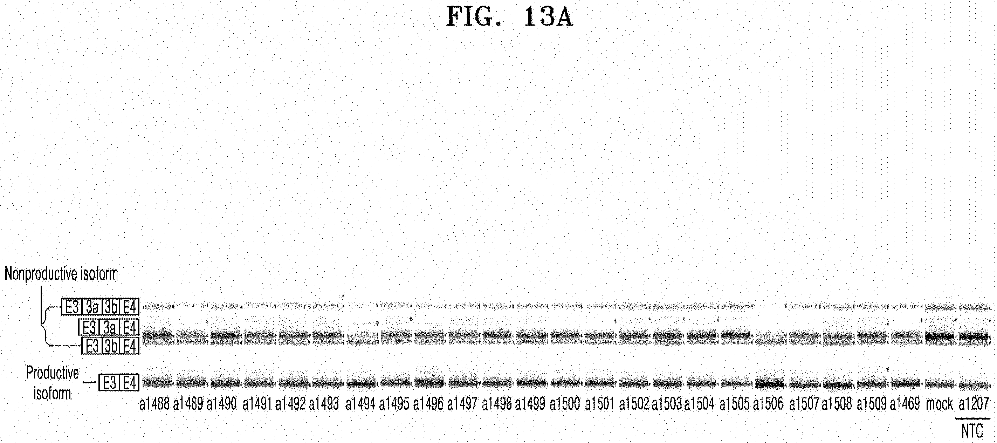

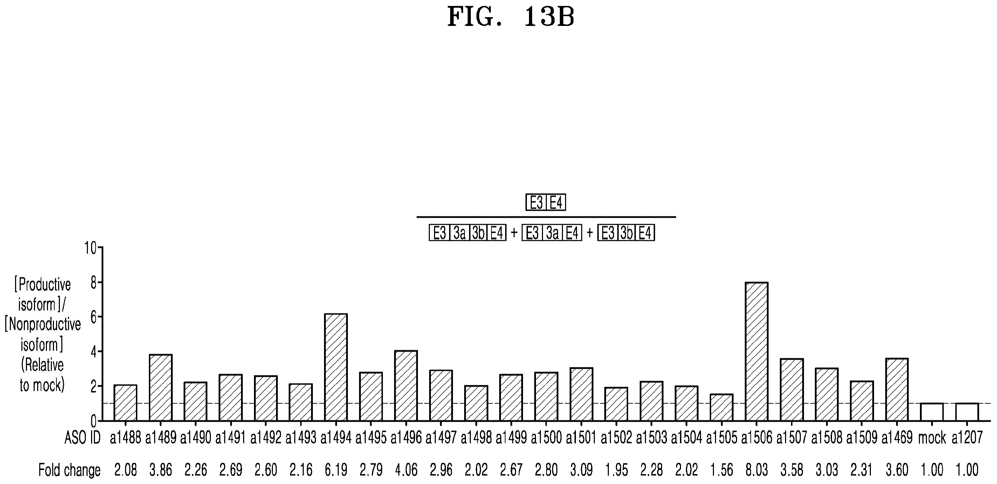

FIGS. 13A and 13B show the screening results of the optimization-designed ASOs for the target region within the *SETD5* 5'-UTR according to an embodiment of the present disclosure. FIG. 13A shows the results of RT-PCR evaluating the changes in the expression of productive and nonproductive isoforms of the *SETD5* gene following treatment with candidate ASOs. FIG. 13B shows the ratio of the productive isoform to the nonproductive isoforms, obtained from the RT-PCR results, relative to the mock group. Mock refers to a control group treated under the same conditions with UPW instead of ASO. In addition, the fold change values relative to the mock group are shown below the ASO IDs in the graph.

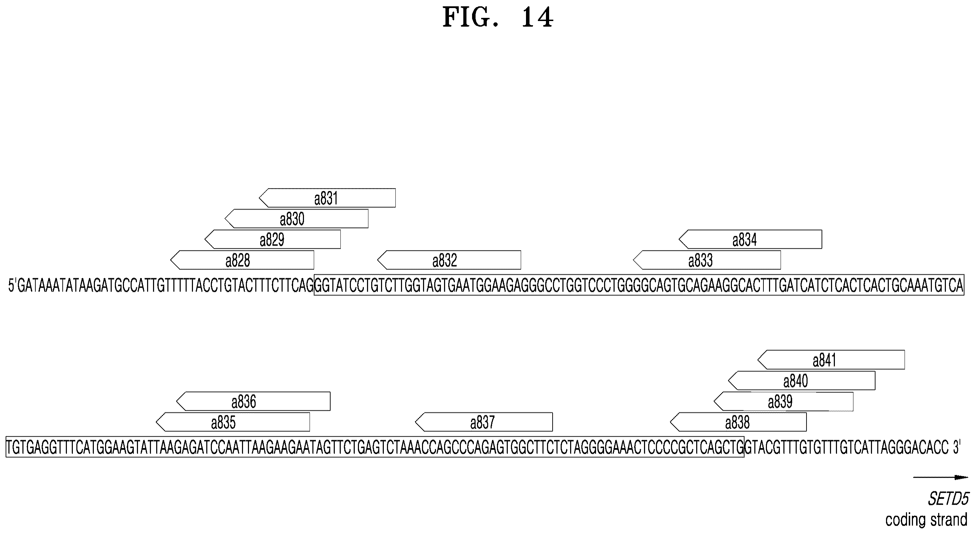

FIG. 14 shows the positions of 20 antisense oligonucleotides designed around exon 3b (193 bp; hg38, chr3:9,433,370-9,433,562), one of the target regions within the *SETD5* 5'-UTR according to an embodiment of the present disclosure. The sequence shown in FIG. 14 corresponds to chr3:9,433,327-9,433,591 based on hg38 reference genome, and is shown in the direction of the plus strand of the genome, corresponding to the coding strand of SETD5. Exon 3b is shaded, and the designed antisense oligonucleotides are represented as triangular-headed boxes at the corresponding positions within exon 3b.

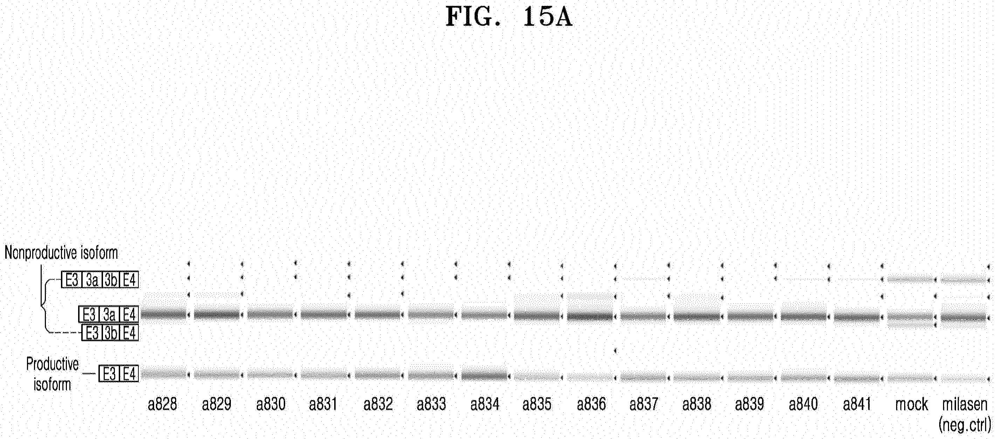

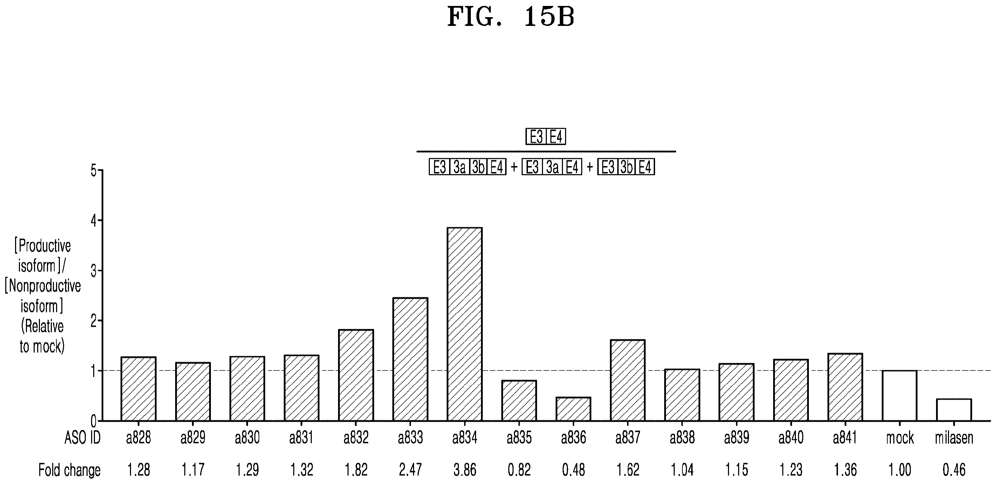

FIGS. 15A and 15B show the screening results of the ASOs designed for exon 3b, which is the target region within the *SETD5* 5'-UTR according to an embodiment of the present disclosure. FIG. 15A shows the results of RT-PCR evaluating changes in the expression of productive and nonproductive isoforms of the *SETD5* gene following treatment with candidate ASOs, and FIG. 15B shows the ratio of the productive isoform to the nonproductive isoforms, obtained from the RT-PCR results, relative to the mock group. Mock refers to a control group treated under the same conditions with UPW instead of ASO. In addition, the fold change values relative to the mock group are shown below the ASO IDs in the graph.

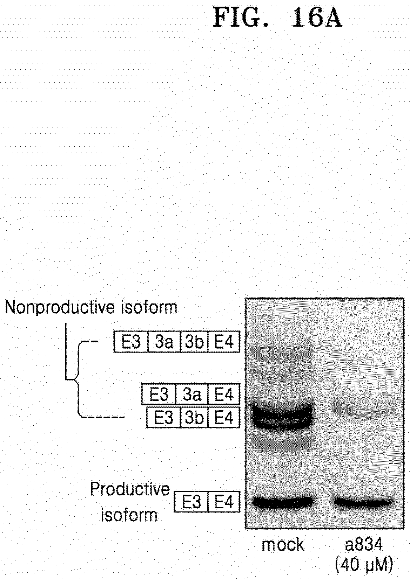

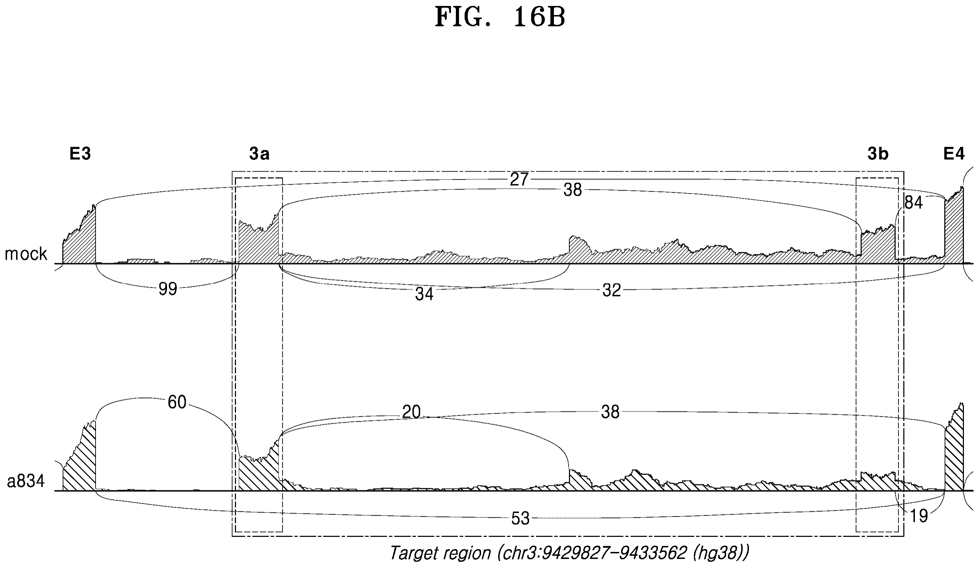

FIGS. 16A and 16B show the changes in the SETD5 isoforms induced by a834 according to an embodiment of the present disclosure in a human cerebral organoid (D170). FIG. 16A shows the results of RT-PCR performed using RNA extracted from human cerebral organoid (D170, cultured for 170 days) after treatment with a834 by gymnotic uptake every three days for two weeks (final concentration of 40 µM),, and FIG. 16B shows the results of RNA-seq performed using RNA extracted following treatment under the same conditions.

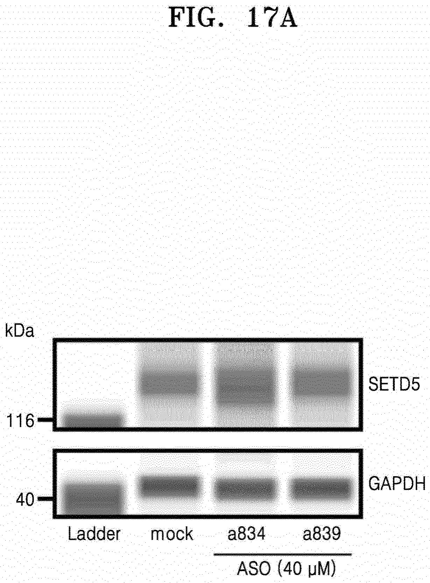

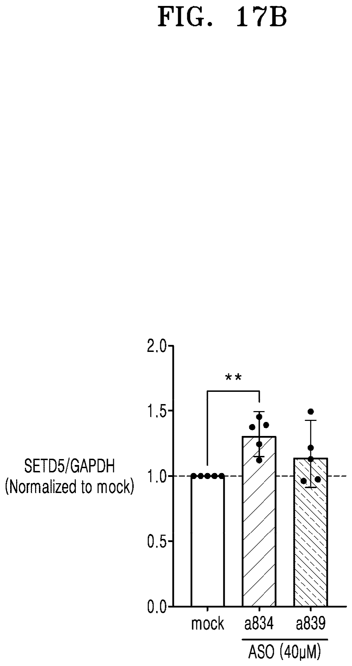

FIGS. 17A and 17B show the increase in SETD5 protein expression induced by ASOs a834, and a839 according to an embodiment of the present disclosure in a human cerebral organoid (D170). FIG. 17A shows the results of Western blot analysis measuring the expression of SETD5 and GAPDH proteins using total cellular proteins extracted from D170 cerebral organoid after treatment with ASOs by gymnotic uptake for two weeks at a final concentration of 40 µM. FIG. 17B presents a graph quantifying the results of FIG. 17A, showing the fold changes in SETD5 protein expression relative to GAPDH compared with the ASO-untreated group (0 µM). Each point represents the result of five independent biological replicate experiments, and the error bars represent the 95 % confidence interval of the geometric mean. Statistical analysis was performed using an unpaired two-tailed t-test with equal variance to evaluate the differences in geometric means between the mock group and each treatment group. As a result, the a834-treated group showed a 1.31-fold increase compared to the mock group, demonstrating a statistically significant difference (P = 0.0044, **). In contrast, the a839-treated group showed a 1.14-fold increase, but the difference was not statistically significant (P = 0.1816, ns).

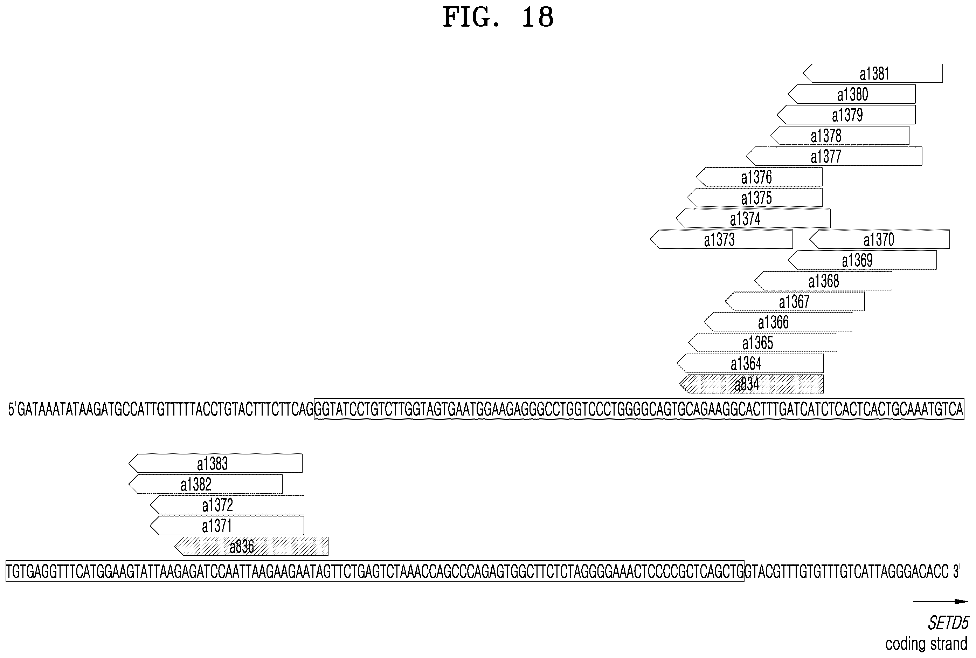

FIG. 18 shows the optimization design of ASOs for exon 3b, which is the target region within the *SETD5* 5'-UTR according to an embodiment of the present disclosure. 16 additional ASOs were designed based on sequences around a834, whose efficacy was confirmed in the primary screening, and 4 additional ASOs were designed around a836, which showed the lowest ratio of productive isoform to nonproductive isoforms of the *SETD5* gene, to be used as controls. The target region, which includes the target exon and its flanking sequences, corresponds to chr3:9,433,327-9,433,591 on the hg38 reference genome, and is shown in the direction of the plus strand of the genome, which corresponds to the coding strand of SETD5.

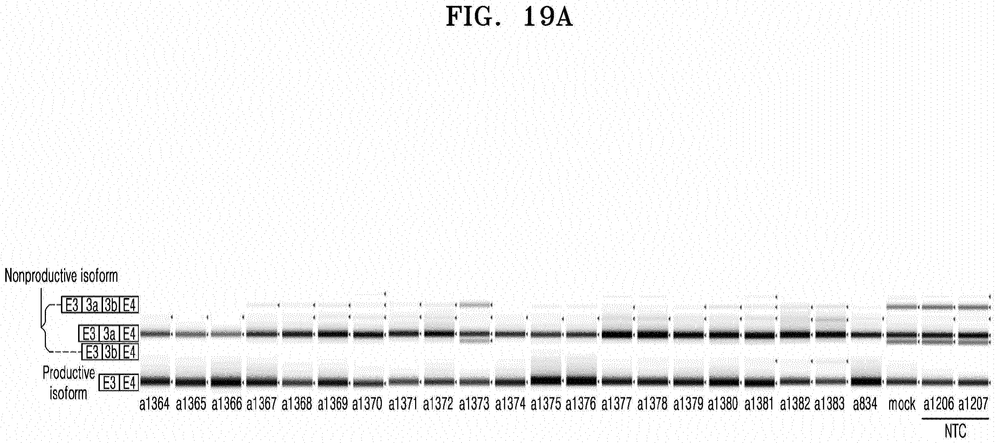

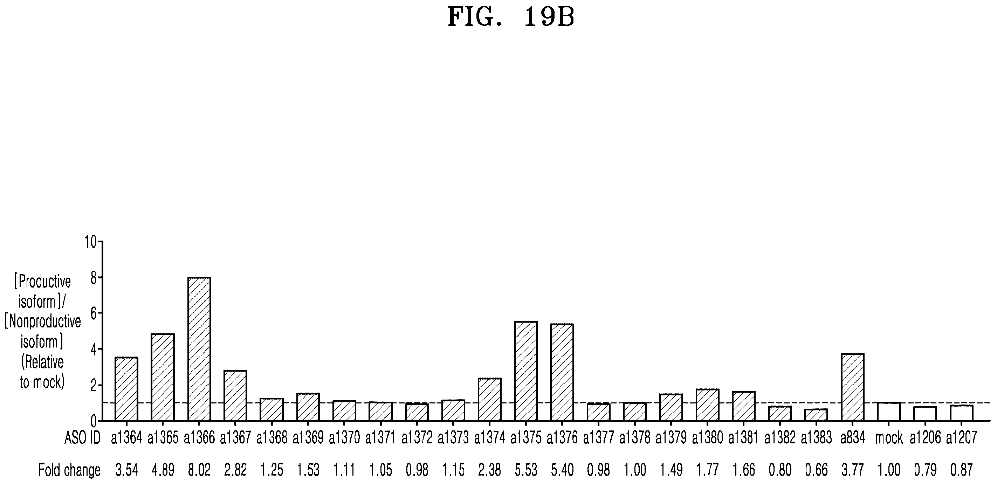

FIGS. 19A and 19B show the screening results of the optimization-designed ASOs for the target region within the *SETD5* 5'-UTR according to an embodiment of the present disclosure. FIG. 19A shows the results of RT-PCR evaluating the changes in the expression of productive isoform and nonproductive isoforms of the *SETD5* gene following treatment with candidate ASOs, and FIG. 19B shows the ratio of productive isoform to nonproductive isoforms relative to the mock group, based on the RT-PCR results. Mock refers to a control group treated under the same conditions with UPW instead of ASO. In addition, the fold change values relative to the mock group are shown below the ASO IDs in the graph.

### Detailed Description

The present disclosure will become apparent with reference to embodiments described in detail below in conjunction with the accompanying drawings. However, the present disclosure is not limited to embodiments below, but will be implemented in various forms. The present embodiments only serve to complete the disclosure of the present disclosure, and are provided to completely inform the scope of the disclosure to ordinary skill in the art to which the present disclosure pertains, and the present disclosure is defined by the claims.

### Example 1. Identification of target regions within SETD5 5'-UTR

### 1.1. Identification of target regions within SETD5 5'-UTR through reanalysis of human neuronal polysome profiling data

Polysome profiling data (GEO accession number: GSE100007) were analyzed, which were obtained by performing RNA-seq on each fraction separated according to the number of ribosomes bound to mRNAs (translation efficiency) in human neuronal cells cultured for 50 days (PMID: 29141229). Isoforms with high translation efficiency were predominantly detected in fractions bound to multiple ribosomes (classified as high polysome), whereas isoforms with low translation efficiency were mainly found in fractions bound to few ribosomes (classified as low polysome). FIG. 1 shows polysome profiling data. Analysis of the polysome profiling data revealed that when exon 3b, present within the 5'-UTR of the *SETD5* gene (Gene ID: 55209, RefSeq accession number: NM_001080517.3), was included through splicing, translation efficiency decreased, indicating the presence of a translation inhibitory element in the corresponding region.

The relative expression levels of isoforms including or excluding exon 3b were quantified in the low polysome fractions and the high polysome fractions from the polysome profiling data of the human neuronal cells cultured 50 days. The results are shown in FIG. 2. Nonproductive isoforms refer to mRNA isoforms that are spliced to include exon 3b, resulting in low translation efficiency, whereas productive isoforms refer to mRNA isoforms that are spliced to exclude exon 3b, resulting in high translation efficiency. From monosome to high polysome fractions, the expression of isoforms in which exon 3b was excluded through splicing (skipping isoform, solid line with open circles) increases, while the proportion of isoforms including exon 3b (inclusion isoform, solid line with open squares) decreases. This indicates that isoforms including exon 3b are a nonproductive type with low translation efficiency, whereas isoforms excluding exon 3b are a productive type that is predominantly present in fractions with high translational activity.

Therefore, when exon 3b is excluded from the target region (i.e., a region including exon 3a and/or exon 3b within the *SETD5* 5'UTR) through splicing modulation, expression of productive *SETD5* isoforms can be promoted, thereby increasing the level of normal SETD5 protein. This approach may be utilized for the treatment of neurodevelopmental disorders such as SETD5 syndrome. FIG. 3 schematically illustrates this therapeutic strategy. By selectively modulating splicing using antisense oligonucleotides targeting a region containing a translation inhibitory element within the 5'-UTR, the generation of nonproductive isoforms containing the exon with the translation inhibitory element can be reduced, while simultaneously increasing the production of productive isoforms lacking this exon, thereby increasing translation efficiency. Isoforms that include the target region between exon 3 and exon 4 of *SETD5* pre-mRNA are considered nonproductive isoforms. In this case, translation via mORF2 (main open reading frame 2) can generate a protein with a partially truncated N-terminus relative to the full-length protein of the MANE (matched annotation from NCBI and EMBL-EBI) transcript, while retaining its main functions. However, translation efficiency via mORF2 is low because it is disrupted by an uORF (upstream open reading frame) starting in exon 3. Conversely, when the target region is excluded, the isoform is classified as productive. In this case, translation occurs via mORF1, producing normal full-length proteins without being impeded by the aforementioned uORF, resulting in high translation efficiency.

### 1.2. Expression level of target region in brain tissue

Using GTEx RNA-seq dataset (phs000424.v10.p2), we examined the inclusion of the target region (hg38, chr3:9,429,827-9,433,562) within the *SETD5* 5'-UTR after splicing across various human tissues. The results are shown in FIG. 4. It was found that, compared with other tissues, a higher proportion of mRNAs include the target region within the *SETD5* 5'-UTR in brain tissue. Exon numbering was based on the SETD5 MANE transcript NM_001080517.3.

Furthermore, we analyzed the human brain long-read RNA-seq dataset (PRJDB15555) to calculate the abundance ratio of each isoform in the region surrounding the previously defined target region (hg38, chr3:9,429,827-9,433,562) within the *SETD5* 5'-UTR. The dataset used for the analysis consisted of postmortem samples from 4 individuals (temporal cortex, hypothalamus, and cerebellum; a total of 12 RNA-seq libraries), which were combined to calculate the read counts for each isoform within the target region. Then, the ratio of the read counts for each isoform to the total read counts of all isoforms was calculated and expressed as abundance (%). The results are shown in panel A of FIG. 5. *The SETD5 MANE transcript, NM_001080517.3 contains the 5'-UTR corresponding to isoform 1.*

The FramePool model (PMID: 33970899) was used to predict translational activity of each isoform. This model estimates the average number of ribosomes bound per mRNA based on the 5'-UTR sequence. Through this, it was confirmed that the translational activity may vary depending on whether specific exons within the *SETD5* 5'UTR are included in the mature mRNA. The analysis predicted that the translational activity would increase and a full-length protein could be produced when either exon 3a or exon 3b of SETD5 is skipped, or when both exons are simultaneously skipped. The results are shown in panel B of FIG. 5. Based on this analysis, exon 3a and exon 3b were identified as target exons, and the region within the *SETD5* 5'UTR containing exon 3a and/or exon 3b was defined as the target region of the present disclosure.

To measure the translational activity of each isoform, four *SETD5* 5'UTR isoforms (isoforms 1, 2, 3, and 5), distinguished by the inclusion or exclusion of exon 3a or exon 3b, were synthesized, and then inserted upstream of the coding sequence (CDS) of the NanoLuc luciferase gene in the NanoLuc reporter vector (pNL1.1 PGK vector, Promega). Isoform 4 was excluded from the analysis because, although its 5'UTR structure is identical to isoform 1, it includes exon 3c, which introduces an early termination within the ORF, making it a nonproductive isoform. The synthesized vectors were transfected into HEK293T cells using Lipofectamine^{™} 3000(Invitrogen) together with a firefly luciferase control vector (pGL4.54[luc2/TK] vector, Promega) at a ratio of 1:4 (total 500 ng). 48 hours after transfection, a Dual-Luciferase Assay (Promega) was performed, and NanoLuc activity was normalized to the firefly luciferase signal. The results are shown in panel D of FIG. 5. Panel D of FIG. 5 shows the relative translational activity of each isoform with isoform 1 as the reference, based on four biological replicates, wherein each biological replicate was summarized as the geometric mean of three technical replicates. Statistical analysis was performed using an unpaired two-tailed equal variance t-test to compare the geometric means of isoform 1 and the other isoforms.

Based on these results, isoform 1 was defined as a productive isoform with high translation efficiency, whereas isoforms 2 to 5, each containing exon 3a, exon 3b, or exon 3c, were defined as nonproductive isoforms with low translation efficiency.

### 1.3. Splicing modulation strategy for SETD5 gene using ASO

Polysome profiling and translation prediction model analysis confirmed that exon 3a, exon 3b, and exon 3c within the *SETD5* 5'UTR act as translation inhibitory elements and affect translation activity of the SETD5. Based on this, a selective splicing strategy was established to enhance SETD5 translational activity by using ASOs targeting these translation inhibitory elements to induce their exclusion from the *SETD5* 5'UTR. By using ASOs to selectively skip one or more target exons (3a, 3b, 3c) of the *SETD5* gene, the expression of nonproductive isoform (such as isoform 2) can be reduced, while the production of productive isoform with high translation efficiency (such as isoform 1) can be increased, thereby enhancing the generation of functional proteins from the SETD5 gene, which can be utilized as a strategy to increase SETD5 protein levels.

### Example 2. Antisense oligonucleotide (ASO) targeting exon 3a

ASOs targeting exon 3a (225 bp; hg38, chr3:9,429,827-9,430,051: SEQ ID NO: 173), which is a translation inhibitory element within the 5'-UTR of the SETD5 pre-mRNA, were designed and screened to enhance expression of the *SETD5* gene as observed in Example 1.

### 2.1. ASO design

A total of 20 ASOs were designed around exon 3a, one of target exons. FIG. 6 shows a schematic diagram of the ASO design. A target region (hg38, chr3:9,429,808-9,430,087), including the target exon 3a and its flanking sequences, is indicated in the plus strand orientation of the genome and the coding strand orientation of SETD5. These ASOs are also expected to increase the production of productive isoforms by inducing skipping of not only exon 3a but also exon 3c.

Table 1 shows the sequence information of the target sequences and the ASO sequences of the designed ASOs.

**[Table 1]**

| **ASO ID** | **Target sequence (5' to 3')** | **SEQ ID NO:** | **ASO sequence (5' to 3')** | **SEQ ID NO:** |
|---|---|---|---|---|
| **a1168** | **GCTTACAGAAAGGAAGTGGAA** | **1** | **TTCCACTTCCTTTCTGTAAGC** | **22** |
| **a1469** | **ACAGAAAGGAAGTGGAAGCC** | **2** | **GGCTTCCACTTCCTTTCTGT** | **23** |
| **81470** | **CCTACCTTTCAAGACGAAGT** | **3** | **ACTTCGTCTTGAAAGGTAGG** | **24** |
| **a1471** | **CCTTTCAAGACGAAGTGGCA** | **4** | **TGCCACTTCGTCTTGAAAGG** | **25** |
| **a1472** | **AAGACGAAGTGGCACTACAC** | **5** | **GTGTAGTGCCACTTCGTCTT** | **26** |
| **a1473** | **CGAAGTGGCACTACACCAG** | **6** | **CTGGTGTAGTGCCACTTCG** | **27** |
| **a1474** | **ACACCAGGATGTGAAAGTCC** | **7** | **GGACTTTCACATCCTGGTGT** | **28** |
| **a1475** | **CAGGATGTGAAAGTCCAGCG** | **8** | **CGCTGGACTTTCACATCCTG** | **29** |
| **a1476** | **ATGTGAAAGTCCAGCGAAAG** | **9** | **CTTTCGCTGGACTTTCACAT** | **30** |
| **a1477** | **GAAAGTCCAGCGAAAGAGAC** | **10** | **GTCTCTTTCGCTGGACTTTC** | **31** |
| **a1478** | **GTCCAGCGAAAGAGACCCAA** | **11** | **TTGGGTCTCTTTCGCTGGAC** | **32** |
| **a1479** | **CCCAGAAGCCAAGTCTATTC** | **12** | **GAATAGACTTGGCTTCTGGG** | **33** |
| **a1480** | **CAGAAGCCAAGTCTATTCCA** | **13** | **TGGAATAGACTTGGCTTCTG** | **34** |
| **a1481** | **GAAGCCAAGTCTATTCCAGG** | **14** | **CCTGGAATAGACTTGGCTTC** | **35** |
| **a1462** | **GCCAAGTCTATTCCAGGCAG** | **15** | **CTGCCTGGAATAGACTTGGC** | **36** |
| **a1483** | **GGATTAGGGTCTGGGCTGG** | **16** | **CCAGCCCAGACCCTAATCC** | **37** |
| **a1484** | **CTTGAGTGAGGTGGGTGTTG** | **17** | **CAACACCCACCTCACTCAAG** | **38** |
| **a1485** | **GTGAGGTGGGTGTTGCTCTA** | **18** | **TAGAGCAACACCCACCTCAC** | **39** |
| **a1486** | **GGTGGGTGTTGCTCTAATTC** | **19** | **GAATTAGAGCAACACCCACC** | **40** |
| **a1487** | **GTTGCTCTAATTCCCCTGTT** | **20** | **AACAGGGGAATTAGAGCAAC** | **41** |
| **a1207** | **CATATTGCGCGTATAGTCGC** | **21** | **GCGACTATACGCGCAATATG** | **42** |

All nucleosides of the designed ASOs were modified with 2'-O-methoxyethyl (2'-MOE), all internucleoside linkages were phosphorothioate (PS), and all cytosines were replaced with 5'-methylcytosines. a1207 was used as a non-targeting control ASO (NTC ASO) for the control group.

### 2.2. ASO screening by RT-PCR

To evaluate the relative expression levels of isoforms containing the target region of the *SETD5* gene by RT-PCR, forward and reverse primers were designed on exon 3 and exon 4, respectively, located on either side of the target region, based on the human genome (hg38) RefSeq transcript NM_001080517.3, as described below. The positions and sequences of the primers are shown in FIG. 7A.
Forward (F) primer 5' - ACATGTTGGATGAGGCTCTG (SEQ ID NO: 163)
Reverse (R) primer 5' - CTCTTCTCATTAACTGCTGGACTA (SEQ ID NO: 164)

In addition, RT-PCR screening was performed to select ASOs that induce splicing to exclude the target region within the 5'-UTR of SETD5 pre-mRNA, thereby increasing the production of productive isoforms that lead to normal SETD5 protein expression. The results are shown in FIGS. 7B and 7C.

The 20 candidate ASOs designed in Section 2.1 and control ASO a1207 (non-targeting control, NTC) were introduced into U2OS cells at a final concentration of 40 nM using Lipofectamine^{™} 3000(Invitrogen). 48 hours after ASO treatment, RNA was extracted and RT-PCR was performed using the primers designed in Section 2.1 to evaluate changes in the expression of productive and nonproductive isoforms of the SETD5 gene in response to each candidate ASO. The RT-PCR results are shown in FIG. 7B. Mock refers to a control group treated under the same conditions with ultra pure water (UPW) instead of ASO. To assess the increase in productive isoforms induced by ASO, three biological replicate experiments were performed. The relative ratio of productive to nonproductive isoforms was calculated from the RT-PCR results and compared to the mock group. The results are shown in FIG. 7C. Error bars represent the 95 % confidence interval of the geometric mean, and each point in the graph represents an individual biological replicate. In addition, the fold change values relative to the mock group are shown below the ASO IDs in the graph.

Among the 20 candidate ASOs, all except a1483 and a1487 increased the ratio of productive to nonproductive isoforms compared to the control ASO group and the mock group. In particular, a1469 (SEQ ID NO: 23), a1471(SEQ ID NO: 25), a1472(SEQ ID NO: 26), and a1474(SEQ ID NO: 28) significantly increased the ratio of the productive isoform.

### 2.3. Changes in SETD5 productive isoform expression induced by candidate ASOs in U2OS cells

Six candidate ASOs that showed more than a threefold splicing effect compared to mock (a1469, a1470, a1471, a1472, a1473, and a1474), as well as a1487, which decreased the proportion of productive isoforms relative to mock, and the control ASO a1207, were transfected into U2OS cells as described in Section 2.2. 48 hours after treatment, RNA was extracted, and RT-qPCR was performed using primers and probes targeting productive SETD5 isoform and all isoforms of GAPDH. The positions and sequences of the primers and probes used for RT-qPCR are shown in FIG. 8A. Expression levels under each condition were measured using a primer-probe set specific for the productive isoform of the *SETD5* gene, while a GAPDH-specific primer-probe set was used to measure GAPDH as an internal control in all groups.

The RT-qPCR results are shown in FIG. 8B. The RT-qPCR results showed that 6 candidate ASOs increased the productive isoform expression by at least twofold compared with the mock group, whereas a1487 reduced the productive isoform expression.

### 2.4. Changes in nuclear SETD5 protein expression induced by a1469 treatment in U2OS cells

Immunofluorescence analysis was performed in U2OS cells treated with a1469, one of the candidate ASOs, as described in Section 2.3, to confirm nuclear localization of SETD5 protein.

Specifically, U2OS cells were fixed with 4 % paraformaldehyde (PFA) and permeabilized with PBS containing 0.3 % Triton X-100. Subsequently, the cells were blocked in a solution containing 5 % FBS, 0.02 % sodium azide, and 0.3 % Triton X-100, and then incubated overnight at 4°C with an anti-SETD5 antibody (PA577197, Thermo Fisher Scientific, 1:1000). After washing, the cells were incubated with Alexa Fluor-conjugated secondary antibody (Invitrogen, 1:1000), and nuclei were stained with 4',6-diamidino-2-phenylindole (DAPI). Images were acquired at 20x magnification and processed to a final size of 165 x 180 pixels (0.3225 µm/pixel, scale bar = 10 µm) for analysis. The results of the immunofluorescence analysis are shown in FIG. 9A. SETD5 was stained with a green-fluorescently labeled primary antibody, and nuclei were stained with DAPI to appear blue. Nuclear localization of SETD5 was confirmed in merged image of the two channels.

Additionally, U2OS cells were treated under the same conditions with PBS only (mock), a1469 (200 nM, candidate ASO), or a1207 (200 nM, control ASO) for 48 hours. Nuclear proteins were then extracted using NE-PER^{™} Nuclear and Cytoplasmic Extraction Reagents (Invitrogen), and SETD5 protein expression was analyzed by capillary-based Western blot. Lamin B1 was used as a loading control group, the SETD5 antibody was MBS8245298 (Mybiosource, 1:12), and the Lamin B1 antibody was ab16048 (abcam, 1:1000). The results are shown in FIGS. 9B and 9C. Each condition was represented by two bands on the membrane, indicating two technical replicates obtained from independent samples. Statistical analysis showed that SETD5 protein expression in the a1207-treated group was not significantly different from the mock group (ns, P = 0.1281), whereas treatment with a1469 significantly increased SETD5 protein expression compared to the mock group (*, P = 0.0146).

### 2.5. Effect of a1469 treatment on SETD5 splicing in mouse brain

Two-day old C57BL/6N mice were administered a1469 (20 µg) or control ASO a1207 (20 µg) via intracerebroventricular (ICV) injection (left ventricle, 2 µl). A mock group, injected with PBS without ASO (mock, 0 µg) was included as a negative control. 7 or 10 days after ASO administration, brains were harvested, and coronal sections (2 mm thick) were prepared from the 2-4 mm anterior region relative to the lambda (λ). RNA was then extracted from the cerebral cortex. Subsequently, RT-PCR was performed to assess the relative expression levels of isoforms containing the target region of the *SETD5* gene.

For RT-PCR, forward and reverse primers were designed on exon 2 and exon 3, respectively, which flank the target region, based on the mouse genome (mm39) RefSeq transcript NM_028385.1, as described below. The positions and sequence information of the primers are shown in FIG. 10A:
Forward (F) primer: 5'-AATTGGACCCCGTGATTCCC (SEQ ID NO: 171)
Reverse (R) primer: 5'-GTCCTCGACACCCATGC (SEQ ID NO: 172).

The results of RT-PCR, which assessed changes in the expression of productive and non-productive isoforms of the *SETD5* gene, are shown in FIGS. 10B and 10C. The target region of a1469 was confirmed to be conserved in mice. FIG. 10C shows the relative ratio of productive to nonproductive isoforms, calculated from the RT-PCR results, and compared to the mock group. Each point represents an individual measurement from mice at 7 days (n=2) and 10 days(n=2) after ASO administration (total n=4). Error bars indicate the 95 % confidence interval of the geometric mean. Statistical analysis was performed using an unpaired two-tailed equal variance t-test to compare the geometric means between the mock group and each treatment group. The analysis showed that the a1469-treated group exhibited a significant difference compared to the mock group (***, P < 1E-3; a1469, P = 0.0004), whereas the a1207-treated group did not show a significant difference from the mock group (P = 0.2795).

### 2.6. SETD5 protein expression changes induced by a1469 treatment in mouse brain

Two-day old C57BL/6N mice were administered a1469 (20 µg) or control ASO a1207 (20 µg) via intracerebroventricular (ICV) injection (left ventricle, 2 µl). A mock group, injected with PBS without ASO (mock, 0 µg) was included as a negative control. 10 days after ASO administration, brains were collected, and coronal sections (2 mm thick) corresponding to the 0-2 mm anterior region from the lambda (λ) reference point were prepared. Nuclear proteins were then extracted from the cerebral cortex and analyzed by Western blot. Nuclear proteins were then extracted using NE-PER^{™} Nuclear and Cytoplasmic Extraction Reagents(Invitrogen), and Lamin B1 was used as a loading control. SETD5 antibody (PA577197, Thermo Fisher Scientific, 1:750) and Lamin B1 antibody (ab16048, Abcam, 1:1000) were used. The Western blot results are shown in FIGS. 11A and 11B. In FIG. 11A, each condition is shown with two bands on the membrane, representing independent biological replicates (n=2). FIG. 11B quantifies the Western blot results and presents the fold changes in SETD5 protein expression relative to Lamin B1 compared to the mock group. In the graph, each condition is presented as the mean ± SD of values from the biological replicate experiments (n=2).

### 2.7. Assessment of a1469 toxicity in mouse brain

In vivo toxicity was assessed in mice by administering ASOs targeting the 5'-UTR of SETD5 pre-mRNA.

Two-day old C57BL/6N mice were administered a1469 (20 to 30 µg) or control ASO a1207 (20 to 30 µg) via ICV (left ventricle, 2 µl). A mock group, injected with PBS without ASO (0 µg) was included as a negative control. Survival was monitored daily for 7 days after administration, and the survival rate was calculated as the ratio of the number of surviving animals at day 7 to the number of animals administered ([number of surviving animals at day 7]/[number of administered animals]). The toxicity of the administered ASOs was then assessed based on the survival rate.

Table 2 shows control ASOs used for toxicity assessment.

**[Table 2]**

| ASO ID | Target sequence (5' to 3') | SEQ ID NO: | ASO sequence (5' to 3') | SEQ ID NO: |
|---|---|---|---|---|
| a386 (toxic ASO) | CGAGAAACCCTACATCTG CA | 43 | TGCAGATGTAGGGTTT CTCG | 45 |
| a143 (ION-626112) | TGAGTCATAACCAGCCTG GC | 44 | GCCAGGCTGGTTATGA CTCA | 46 |
| a1207 (NTC ASO) | CATATTGCGCGTATAGTC GC | 21 | GCGACTATACGCGCAA TATG | 42 |

a386 (toxic ASO) is an ASO previously shown to have high toxicity, with the chemical formula of T*GCAG*5*8*7*8*5*7*7*7*8*8*TCTC*G; and a143 (ION-626112) is an ASO targeting the long non-coding RNA Malat1, reported to inhibit its function with high activity and high safety, and has a chemical formula of G*CCAG7*6*8*7*7*8*8*5*8*7*ACT*C*A (A=2'MOE-A, C=2'MOE-5'-methyl-C, G=2'MOE-G, T=2'MOE-T, 5=DNA-A, 6=DNA-5'-methyl-C, 7=DNA-G, 8=DNA-T, *=phosphorothioate).

The safety of a1469 was confirmed to be comparable to that of a1207 and a143. The results are shown in Table 3.

**[Table 3]**

| **Condition** | **ASO ID** | **ASO dose** | | | |
|---|---|---|---|---|---|
| | | **0** µ**g** | **20** µ**g** | **25** µ**g** | **30** µ**g** |
| **PBS** | **-** | 12/12 | | | |
| **Control group** | **a386 (toxic ASO)** | | 0/11 | | |
| | **a1207 (NTC ASO)** | | 10/12 | 1/2 | 2/11 |
| | **a143 (ION-626112)** | | 7/10 | | |
| **Experi mental group** | **a1469** | | 10/13 | 2/3 | 4/13 |

### 2.8. Optimization design

Based on the primary screening of ASOs targeting exon 3a, a total of 22 additional ASOs were designed around the target regions of a1469(SEQ ID NO: 23) and a1474(SEQ ID NO: 28), which showed the highest efficacy in increasing the productive isoform of the SETD5. FIG. 12 shows target exon 3a indicated in a box, along with a1469 and a1474 (dot-shaded) and 22 additionally designed ASOs (unshaded), all of which are arranged along the exon. The target region (hg38, chr3:9,429,808-9,430,087), including target exon and its flanking sequences, is shown in the plus strand orientation of the genome and in the coding strand orientation of SETD5, and exon 3a (hg38, chr3:9,429,827-9.430,051, 225 bp), which is the target exon of the designed ASOs, is indicated by a box.

Table 4 shows the ASO target sequences and corresponding ASO sequences designed around the target regions of ASOs that were selected based on their ability to increase productive SETD5 isoforms.

**[Table 4]**

| **ASO ID** | **Target sequence (5' to 3')** | **SEQ ID NO:** | **ASO sequence (5' to 3')** | **SEQ ID NO:** |
|---|---|---|---|---|
| **a1488** | **TTACAGAAAGGAAGTGGAAGC** | **47** | **GCTTCCACTTCCTTTCTGTAA** | **69** |
| **a1489** | **TTACAGAAAGGAAGTGGAAGCC** | **48** | **GGCTTCCACTTCCTTTCTGTAA** | **70** |
| **a1490** | **TACAGAAAGGAAGTGGAAGC** | **49** | **GCTTCCACTTCCTTTCTGTA** | **71** |
| **a1491** | **TACAGAAAGGAAGTGGAAGCC** | **50** | **GGCTTCCACTTCCTTTCTGTA** | **72** |
| **a1492** | **TACAGAAAGGAAGTGGAAGCCC** | **51** | **GGGCTTCCACTTCCTTTCTGTA** | **73** |
| **a1493** | **ACAGAAAGGAAGTGGAAGC** | **52** | **GCTTCCACTTCCTTTCTGT** | **74** |
| **a1494** | **ACAGAAAGGAAGTGGAAGCCC** | **53** | **GGGCTTCCACTTCCTTTCTGT** | **75** |
| **a1495** | **CAGAAAGGAAGTGGAAGC** | **54** | **GCTTCCACTTCCTTTCTG** | **76** |
| **a1498** | **CAGAAAGGAAGTGGAAGCC** | **55** | **GGCTTCCACTTCCTTTCTG** | **77** |
| **a1497** | **CAGAAAGGAAGTGGAAGCCC** | **56** | **GGGCTTCCACTTCCTTTCTG** | **78** |
| **a1498** | **AGAAAGGAAGTGGAAGC** | **57** | **GCTTCCACTTCCTTTCT** | **79** |
| **a1499** | **AGAAAGGAAGTGGAAGCC** | **58** | **GGCTTCCACTTCCTTTCT** | **80** |
| **a1500** | **AGAAAGGAAGTGGAAGCCC** | **59** | **GGGCTTCCACTTCCTTTCT** | **81** |
| **a1501** | **GAAAGGAAGTGGAAGCC** | **60** | **GGCTTCCACTTCCTTTC** | **82** |
| **a1502** | **GAAAGGAAGTGGAAGCCC** | **61** | **GGGCTTCCACTTCCTTTC** | **83** |
| **a1503** | **AAAGGAAGTGGAAGCC** | **62** | **GGCTTCCACTTCCTTT** | **84** |
| **a1504** | **AAAGGAAGTGGAAGCCC** | **63** | **GGGCTTCCACTTCCTTT** | **85** |
| **a1505** | **AAGGAAGTGGAAGCCC** | **64** | **GGGCTTCCACTTCCTT** | **86** |
| **a1506** | **GTGGCACTACACCAGGATGT** | **65** | **ACATCCTGGTGTAGTGCCAC** | **87** |
| **a1507** | **GGCACTACACCAGGATGTGA** | **66** | **TCACATCCTGGTGTAGTGCC** | **88** |
| **a1508** | **CACTACACCAGGATGTGAAA** | **67** | **TTTCACATCCTGGTGTAGTG** | **89** |
| **a1509** | **CTACACCAGGATGTGAAAGT** | **68** | **ACTTTCACATCCTGGTGTAG** | **90** |

All nucleotides in the designed ASOs contained 2'MOE modifications, all internucleoside linkages were phosphorothioate (PS), and all cytosine bases were replaced with 5'-methylcytosine.

### 2.9. Screening of optimized ASOs using RT-PCR

ASO a1469 with the highest efficacy in the primary screening, 22 candidate ASOs derived from the optimization design, and a single control ASO a1207 were introduced into U2OS cells using Lipofectamine^{™} 3000 (Invitrogen) at a final concentration of 40 nM. 48 hours after ASO transfection, RNA was extracted and subjected to RT-PCR, and the changes in expression of productive and nonproductive *SETD5* isoforms were assessed for each candidate ASO treatment. Mock refers to a control group treated under the same conditions with UPW instead of ASO. The results are shown in FIGS. 13A and 13B .

Based on the RT-PCR results of FIG. 13A, FIG. 13B shows the results of comparing the ratio of productive *SETD5* isoform to nonproductive *SETD5* isoforms relative to the mock group. In addition, the fold change relative to the mock group is indicated below each ASO ID in the graph. In this optimization screening, a1489, a1494, a1496, and a1506 were found to increase productive isoforms more effectively than, or similarly to, a1469, which was the most effective in the primary screening.

### Example 3. ASO targeting exon 3b

ASOs targeting exon 3b (193 bp; hg38, chr3:9,433,327-9,433,591: SEQ ID NO: 174), which is a translation inhibitory site within the 5'-UTR of the SETD5 pre-mRNA, were designed and screened to enhance expression of the *SETD5* gene as observed in Example 1.

### 3.1. ASO design

A total of 14 ASOs were designed around exon 3b (193 bp; hg38, chr3:9,433,370-9,433,562, shaded square), the target exon of the *SETD5* gene. FIG. 14 shows a schematic diagram of the ASO design. A target region (hg38, chr3:9,433,327-9,433,591), including the target exon and its flanking sequences, is indicated in the plus strand orientation of the genome and the coding strand orientation of SETD5.

Table 5 shows the sequence information of the target sequences and the ASO sequences of the ASOs designed for exon 3b.

**[Table 5]**

| **ASO ID** | **Target sequence {5' to 3'}** | **SEQ ID_NO:** | **ASO: sequence {5' to 3'}** | **SEQ ID NO:** |
|---|---|---|---|---|
| **a828** | **TTTTACCTGTACTTTCTTCAG** | **91** | **CTGAAGAAAGTACAGGTAAAA** | **106** |
| **a829** | **CCTGTACTTTCTTCAGGGTA** | **92** | **TACCCTGAAGAAAGTACAGG** | **107** |
| **a830** | **GTACTTTCTTCAGGGTATCCT** | **93** | **AGGATACCCTGAAGAAAGTAC** | **108** |
| **a831** | **TTCTTCAGGGTATCCTGTCT** | **94** | **AGACAGGATACCCTGAAGAA** | **109** |
| **a832** | **TCTTGGTAGTGAATGGAAGA** | **95** | **TCTTCCATTCACTACCAAGA** | **110** |
| **8833** | **GGCAGTGCAGAAGGCACTTT** | **96** | **AAAGTGCCTTCTGCACTGCC** | **111** |
| **a834** | **GCAGAAGGCACTTTGATCAT** | **97** | **ATGATCAAAGTGCOTTCTSC** | **112** |
| **8835** | **TAAGAGATCCAATTAAGAAGAA** | **98** | **TTCTTCTTAATTGGATCTCTTA** | **113** |
| **a836** | **GAGATCCAATTAAGAAGAATAG** | **99** | **CTATTCTTCTTAATTGGATCTC** | **114** |
| **a837** | **ACCAGCCCAGAGTGGCTTC** | **100** | **GAAGCCACTCTGGGCTGGT** | **115** |
| **a838** | **CGCTCAGCTGGTAGGTTTG** | **101** | **CAAACGTACCAGCTGAGCG** | **116** |
| **a839** | **GCTGGTACGTTTGTGTTTGT** | **102** | **ACAAACACAAACGTACCAGC** | **117** |
| **a840** | **TGGTACGTTTGTGTTTGTCAT** | **103** | **ATGACAAACACAAACGTACCA** | **118** |
| **a841** | **ACGTTTGTGTTTGTCATTAGG** | **104** | **CCTAATGACAAACACAAACGT** | **119** |
| **milasen** | **GCCCTCAACAAGCACTAACATT** | **105** | **AATGTTAGTGCTTGTTGAGGGC** | **120** |

All nucleosides of the designed ASOs were modified with 2'-O-methoxyethyl (2'-MOE), all internucleoside linkages were phosphorothioate (PS), and all cytosines were replaced with 5'-methylcytosine. Milasen, an ASO that targets a gene other than the SETD5, was used as a control.

### 3.2. ASO screening by RT-PCR

The 14 candidate ASOs, which were designed to target exon 3b, and milasen as a control ASO, were introduced into U2OS cells at a final concentration of 40 nM using Lipofectamine^{™} 3000 (Invitrogen). 48 hours after ASO treatment, RNA was extracted and RT-PCR was performed to assess changes in the expression of productive and nonproductive *SETD5* isoforms in response to treatment with each candidate ASO, as described in Section 2.2. The results are shown in FIGS. 15A and 15B . Mock refers to a control group treated under the same conditions with UPW instead of ASO. Based on the RT-PCR results, FIG. 15B shows the results of comparing the ratio of the productive isoform to the nonproductive isoforms of the *SETD5* gene relative to the mock group. ASOs a832, a833, and a834 showed more than a 1.8 fold increase compared to the mock group, with a834 exhibiting the greatest increase.

### 3.3. Changes in SETD5 isoforms induced by a834 in human cerebral organoid(D170)

a834 (candidate ASO), which showed the most potent effect of increasing the productive isoform of the SETD5 gene in Section 3.2, was applied to human cerebral organoid (D170) differentiated from DF19-9-7T induced pluripotent stem cells (iPSCs) and cultured for 170 days, and changes in *SETD5* isoform expression were evaluated. The human cerebral organoid (D170) was treated with a834 via gymnotic uptake at a final concentration of 40 µM every 3 days for 2 weeks, followed by RNA extraction. RT-PCR and RNA-seq were then performed on the extracted RNA. The results are each shown in FIGS. 16A and 16B.

In the control group (mock), nonproductive isoforms were abundantly expressed, whereas their expression was markedly reduced in the a834 treatment group.

### 3.4. Increase in SETD5 protein expression by candidate ASOs in human cerebral organoid (D170)

Human cerebral organoids (D170) were treated with candidate ASOs, a834 and a839, via gymnotic uptake for 2 weeks at a final concentration of 40 µM. Subsequently, total cellular proteins were extracted using RIPA lysis and extraction buffer (Thermo Scientific^{™}), and SETD5 and GAPDH protein expression was measured by capillary Western blot using SETD5 (MBS8245298, Mybiosource; dilution at 1:15) and GAPDH (ab8245, Abcam; dilution at 1:1800) antibodies. The results are shown in FIGS. 17A and 17B . FIG. 17B shows a graph quantifying the Western blot results, showing the fold change in the SETD5 protein expression relative to GAPDH, compared to the ASO-untreated group (0 µM). a834 and a839 increased SETD5 expression relative to the control group (mock), with a834 showing the most pronounced increase.

### 3.5. ASO optimization for exon 3b

16 ASOs were additionally designed around a834, which showed efficacy in the primary screening. Furthermore, as a control group, 4 ASOs were designed around a836, which showed the lowest ratio of productive to nonproductive isoforms of the *SETD5* gene. In FIG. 18, the target region (hg38, chr3:9,433,327-9,433,591), including target exon 3b and its flanking sequences, is shown in the plus strand orientation of the genome and the coding strand orientation of SETD5. The figure also shows a834 and a836 arranged on the target sequences, along with ASOs designed around these sites with subtle differences in length and position.

Table 6 lists the ASOs optimally designed for exon 3b and their target sequences.

**[Table 6]**

| **ASO ID** | **Target sequence (5' to 3')** | **SEQ ID NO:** | **ASO sequence (5' to 3')** | **SEQ ID NO:** |
|---|---|---|---|---|
| **a1364** | **TGCAGAAGGCACTTTGATCAT** | **121** | **ATGATCAAAGTGCCTTCTGCA** | **142** |
| **a1365** | **CAGAAGGCACTTTGATCATCT** | **122** | **AGATGATCAAAGTGCCTTCTG** | **143** |
| **a1366** | **GAAGGCACTTTGATCATCTCA** | **123** | **TGAGATGATCAAAGTGCCTTC** | **144** |
| **a1367** | **GGCACTTTGATCATCTCACT** | **124** | **AGTGAGATGATCAAAGTGCC** | **145** |
| **a1368** | **CTTTGATCATCTCACTCACT** | **125** | **AGTGAGTGAGATGATCAAAG** | **146** |
| **a1369** | **ATCATCTCACTCACTGCAAAT** | **126** | **ATTTGCAGTGAGTGAGATGAT** | **147** |
| **a1370** | **ATCTCACTCACTGCAAATGT** | **127** | **ACATTTGCAGTGAGTGAGAT** | **148** |
| **a1371** | **ATTAAGAGATCCAATTAAGAAG** | **128** | **CTTCTTAATTGGATCTCTTAAT** | **149** |
| **a1372** | **TTAAGAGATCCAATTAAGAAGA** | **129** | **TCTTCTTAATTGGATCTCTTAA** | **150** |
| **a1373** | **CAGTGCAGAAGGCACTTTGA** | **130** | **TCAAAGTGCCTTCTGCACTG** | **151** |
| **a1374** | **TGCAGAAGGCACTTTGATCATC** | **131** | **GATGATCAAAGTGCCTTCTGCA** | **152** |
| **a1375** | **CAGAAGGCACTTTGATCAT** | **132** | **ATGATCAAAGTOCCTTCTG** | **153** |
| **a1376** | **AGAAGGCACTTTGATCAT** | **133** | **ATGATCAAAGTGCCTTCT** | **154** |
| **a1377** | **ACTTTGATCATCTCACTCACTGCAA** | **134** | **TTGCAGTGAGTGAGATGATCAAAGT** | **155** |
| **a1378** | **TTGATCATCTCACTCACTGC** | **135** | **GCAGTGAGTGAGATGATCAA** | **156** |
| **a1379** | **TGATCATCTCACTCACTGCA** | **136** | **TGCAGTGAGTGAGATGATCA** | **157** |
| **a1380** | **ATCATCTCACTCACTGCA** | **137** | **TGCAGTGAGTGAGATGAT** | **158** |
| **a1381** | **CATCTCACTCACTGCAAATG** | **138** | **CATTTGCAGTGAGTGAGATG** | **159** |
| **a1382** | **GTATTAAGAGATCCAATTAAGA** | **139** | **TCTTAATTGGATCTCTTAATAC** | **160** |
| **a1383** | **GTATTAAGAGATCCAATTAAGAAGA** | **140** | **TCTTCTTAATTGGATCTCTTAATAC** | **161** |
| **a1206** | **CGAGCGTGATTAAACTAA** | **141** | **TTAGTTTAATCACGCTCG** | **162** |

All nucleotides in the designed ASOs were modified with 2'MOE, all internucleoside linkages were PS, and all cytosines were replaced with 5'-methylcytosine. ASO a1206 was used as a non-targeting control (NTC).

### 3.6. Screening of optimized ASOs using RT-PCR

ASO a834, which showed the highest efficacy in the primary screening, a total of 20 candidate ASOs derived through optimization design (16 ASOs designed around the target region of a834 and 4 ASOs designed around the target region of a836), and 2 control ASOs (a1206, a1207; NTC), were introduced into U2OS cells at a final concentration of 40 nM using Lipofectamine^{™} 3000 (Invitrogen). 48 hours after ASO introduction, RNA was extracted and RT-PCR was performed. The changes in expression of productive and nonproductive isoforms of the *SETD5* gene were then evaluated for each candidate ASO. Mock refers to a control group treated under the same conditions with UPW instead of ASO. The results are shown in FIGS. 19A and 19B.

Based on the RT-PCR results of FIG. 19A, FIG. 19B presents the ratio of the productive isoform to the nonproductive isoforms of the *SETD5* gene relative to the mock group. In this optimization screening, a1364 to a1366, a1375, and a1376 were found to increase the productive isoform more effectively than a834, which had the highest efficacy in the primary screening, with a1366 showing the most pronounced effect.

## Claims

1. An antisense oligonucleotide targeting a pre-mRNA of the human *SETD5* gene,
the antisense oligonucleotide comprises 13 to 40 consecutive nucleotides and binds to a target region of the SETD5 pre-mRNA by hybridization via at least 13 consecutive Watson-Crick base pairings (A:T or G:C) or wobble base pairings (G:U, I:A, I:C, or I:U), thereby increasing the expression of SETD5,
wherein the target region comprises a sequence selected from the group consisting of SEQ ID NOs: 1 to 15, 17 to 19, 47 to 68, 95 to 97, 100, 121 to 124, 126, 131 to 133, 137, and 138.

2. The antisense oligonucleotide of claim 1, wherein the target region comprises a sequence selected from the group consisting of SEQ ID NOs: 2 to 7, 9, 11, 12, 48, 53, 55, 60, 65 to 67, 97, 121 to 123, 132, and 133.

3. The antisense oligonucleotide of claim 1 or 2, wherein the target region comprises a sequence selected from the group consisting of SEQ ID NOs: 2 to 7, 48, 53, 55, 65, 97, 122, 123, 132, and 133.

4. The antisense oligonucleotide of any one of claims 1 to 3, wherein the target region comprises a sequence selected from the group consisting of SEQ ID NOs: 2, 53, 65, 97, 123, 132, and 133.

5. The antisense oligonucleotide of any one of claims 1 to 4, wherein the antisense oligonucleotide comprises a sequence selected from the group consisting of SEQ ID NOs: 22 to 36, 38 to 40, 69 to 90, 110 to 112, 115, 142 to 145, 147, 152 to 154, 158, and 159.

6. An antisense oligonucleotide targeting a pre-mRNA of the human *SETD5* gene,
the antisense oligonucleotide comprises 13 to 40 consecutive nucleotides and binds to at least 80 % of nucleobases in a target region of the SETD5 pre-mRNA by hybridization via Watson-Crick base pairings (A:T or G:C) or wobble base pairings (G:U, I:A, I:C, or I:U), thereby increasing the expression of SETD5,
wherein the target region comprises a sequence selected from the group consisting of SEQ ID NOs: 1 to 15, 17 to 19, 47 to 68, 95 to 97, 100, 121 to 124, 126, 131 to 133, 137, and 138.

7. The antisense oligonucleotide of claim 6, wherein the target region comprises a sequence selected from the group consisting of SEQ ID NOs: 2 to 7, 9, 11, 12, 48, 53, 55, 60, 65 to 67, 97, 121 to 123, 132, and 133.

8. The antisense oligonucleotide of claim 6 or 7, wherein the target region comprises a sequence selected from the group consisting of SEQ ID NOs: 2 to 7, 48, 53, 55, 65, 97, 122, 123, 132, and 133.

9. The antisense oligonucleotide of any one of claims 6 to 8, wherein the target region comprises a sequence selected from the group consisting of SEQ ID NOs: 2, 53, 65, 97, 123, 132, and 133.

10. The antisense oligonucleotide of any one of claims 6 to 9, wherein the antisense oligonucleotide comprises a sequence selected from the group consisting of 'SEQ ID NOs: 22 to 36, 38 to 40, 69 to 90, 110 to 112, 115, 142 to 145, 147, 152 to 154, 158, and 159.

11. The antisense oligonucleotide of any one of claims 1 to 10, wherein the antisense oligonucleotide comprises at least one modification of a chemically modified sugar moiety, a chemically modified base, and a chemically modified internucleoside linkage, wherein
wherein the chemically modified sugar moiety comprises at least one sugar moiety modified with 2'-O-methyl, 2'-methoxyethoxy, 2'-O-methoxyethyl (2'-MOE), 2'-dimethylaminoethoxy, 2'-dimethylaminoethoxyethoxy, 2'-fluoro, 2'-acetamide, morpholino, locked nucleic acid (LNA), or S-constrained-ethyl (c-ET),
the modified base comprises at least one of 5-methylcytosine, 5-fluorocytosine, and 2-aminopurine base, and
the chemically modified internucleoside linkage comprises a phosphorothioate linkage.

12. The antisense oligonucleotide of any one of claims 1 to 11, wherein the antisense oligonucleotide consists of nucleosides having a 2'-MOE modification and includes phosphorothioate as the internucleoside linkages and 5'-methyl-cytosine for all cytosine (C) bases.

13. The antisense oligonucleotide of any one of claims 1 to 12, wherein the antisense oligonucleotide has one of structures of Formulas (I) to (VII):
G*G*C*T*T*C*C*A*C*T*T*C*C*T*T*T*C*T*G*T Formula (I);
G*G*G*C*T*T*C*C*A*C*T*T*C*C*T*T*T*C*T*G*T Formula (II);
A*C*A*T*C*C*T*G*G*T*G*T*A*G*T*G*C*C*A*C Formula (III);
A*T*G*A*T*C*A*A*A*G*T*G*C*C*T*T*C*T*G*C Formula (IV);
T*G*A*G*A*T*G*A*T*C*A*A*A*G*T*G*C*C*T*T*C Formula (V);
A*T*G*A*T*C*A*A*A*G*T*G*C*C*T*T*C*T*G Formula (VI);
and
A*T*G*A*T*C*A*A*A*G*T*G*C*C*T*T*C*T Formula (VII).
wherein A is 2'MOE-A, C is 2'MOE-5'-methyl-C, G is 2'MOE-G, T is 2'MOE-T, * indicates phosphorothioate (PS), and 2'MOE is 2'-O-methoxyethyl.

14. The antisense oligonucleotide of any one of claims 1 to 13, wherein the antisense oligonucleotide has one of structures of Formulas (I) and (IV):
G*G*C*T*T*C*C*A*C*T*T*C*C*T*T*T*C*T*G*T Formula (I);
and
A*T*G*A*T*C*A*A*A*G*T*G*C*C*T*T*C*T*G*C Formula (IV);
wherein A is 2'MOE-A, C is 2'MOE-5'-methyl-C, G is 2'MOE-G, T is 2'MOE-T, * indicates phosphorothioate (PS), and 2'MOE is 2'-O-methoxyethyl.

15. Use of the antisense oligonucleotide of any one of claims 1 to 14 for treating a disease associated with an abnormal level or abnormal activity of SETD5, wherein the disease is SETD5 syndrome.
